# EUROPEAN PATENT APPLICATION

(11) **EP 4 162 920 A1**
(43) Date of publication of application: **12.04.2023**
(21) Application number: 21838297.6
(22) Date of filing: 09.07.2021
(51) Int. Cl.: A61K 6/30, A61K 6/60, A61K 6/70

(54) **DENTAL ADHESIVE COMPOSITION AND DENTAL MATERIAL**

(30) Priority: 09.07.2020 JP 2020118821; 09.07.2020 JP 2020118822
(71) Applicant: Mitsui Chemicals, Inc., Tokyo 105-7122 (JP)
(72) Inventor: KAKINUMA, Naoyuki, Sodegaura-shi, Chiba 299-0265 (JP)
(74) Representative: Hoffmann Eitle
(86) International application number: PCT/JP2021/025954
(87) International publication number: WO 2022/009986

(57) **Abstract**

A dental adhesive composition containing: a (meth)acrylate compound (1) having an acidic group; and at least one polymerizable compound (2) selected from the group consisting of a cyclopolymerizable compound (2a) and a polymerizable compound (2b) having a cyclic structure and one polymerizable group.

## Description

### Technical Field

The present disclosure relates to a dental adhesive composition and a dental material.

### Background Art

In dental treatment, a synthetic resin molded product or the like is used for the purpose of tooth restoration.

Examples of the material for forming the synthetic resin molded product include a composite resin, a bonding material, and a resin cement.

For example, Patent Document 1 describes a self-adhesive dental composite resin containing an acidic group-containing (meth)acrylic polymerizable monomer (a), a polyfunctional (meth)acrylic polymerizable monomer (b) not containing an acidic group, a photopolymerization initiator (c), fumed silica (d), a long-chain organosilicon compound (e), and a filler (f) (except fumed silica (d)), in which the filler (f) is treated with a surface treatment agent and has an average particle size of 0.01 to 50.0 µm, the surface treatment agent contains a specific silane coupling agent (A) and a specific organosilazane (B), and the long-chain organosilicon compound (e) is a specific compound.

Patent Document 1: Japanese Patent Application Laid-Open No. 2018-145133

### SUMMARY OF INVENTION

### Technical Problem

Among the above materials, for example, a bonding material is used for the purpose of bonding objects to each other.

In dentistry, examples of the object to which a bonding material is bonded include tooth substances (for example, enamel, dentin, etc.), precious metals (for example, gold, palladium, etc.), base metals (for example, zirconia, etc.), and porcelain materials (for example, filler, silica, etc.).

A conventional bonding material is often used as a composition for a dental material, and a composition containing a bonding material has a problem that adhesive performance deteriorates over time during storage.

In Patent Document 1, it has not been studied to obtain a dental adhesive composition which is excellent in adhesiveness in a cured product obtained when cured and has good storage stability.

Among the above materials, for example, a bonding material is used for the purpose of bonding objects to each other.

In dentistry, examples of the object to which a bonding material is bonded include tooth substances (for example, enamel, dentin, etc.), precious metals (for example, gold, palladium, etc.), base metals (for example, zirconia, etc.), and porcelain materials (for example, filler, silica, etc.).

Conventionally, two types of dental bonding materials: aqueous bonding materials and non-aqueous bonding materials, have been used. The aqueous bonding material and the non-aqueous bonding material may be selectively used depending on an object to be bonded.

For example, when the object to be adhered is a porcelain material, a non-aqueous bonding material tends to be used, and when the object to be adhered is a tooth substance, an aqueous bonding material tends to be used.

From the above, for example, a composition exhibiting adhesiveness to both a tooth substance and a porcelain material has been required.

In Patent Document 1, it has not been studied to exhibit adhesiveness to both a tooth substance and a porcelain material.

The problem to be solved by one embodiment of the first embodiment is to provide a dental adhesive composition which is excellent in adhesiveness in a cured product obtained when cured and has good storage stability, and a dental material obtained from the dental adhesive composition.

The problem to be solved by one embodiment of the second embodiment is to provide a dental adhesive composition capable of improving adhesiveness to a tooth substance and a porcelain material in a cured product obtained when cured.

Further, the problem to be solved by another embodiment of the second embodiment is to provide a dental material having excellent adhesiveness to a tooth substance and a porcelain material.

### Solution to Problem

Means for solving the problem of the first embodiment includes the following aspects.
<1> A dental adhesive composition, containing: a (meth)acrylate compound (1) having an acidic group; and at least one polymerizable compound (2) selected from the group consisting of a cyclopolymerizable compound (2a) and a polymerizable compound (2b) having a cyclic structure and one polymerizable group.
<2> The dental adhesive composition according to <1>, in which the cyclopolymerizable compound (2a) includes an acrylic acid ester compound (2a-1) having one allyloxy group, and the polymerizable compound (2b) includes a (meth)acrylate compound (2b-1) having a cyclic structure having 3 to 12 carbon atoms and one (meth)acryloyloxy group.
<3> The dental adhesive composition according to <1> or <2>, in which the polymerizable compound (2) is at least one selected from the group consisting of a compound represented by the following Formula (2a-1a) and a compound represented by the following Formula (2b-1a).

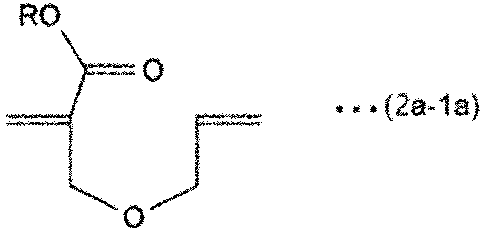

In Formula (2a-1a), R represents a hydrogen atom or a substituted or unsubstituted hydrocarbon group having 1 to 20 carbon atoms.

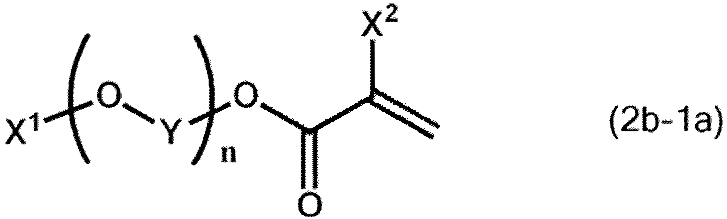

In Formula (2b-1a), X¹ represents an alicyclic, aromatic, or heterocyclic ring having 4 to 10 carbon atoms, or an alkyl group having 1 to 3 carbon atoms substituted with an alicyclic, aromatic, or heterocyclic ring having 4 to 10 carbon atoms, X² represents a hydrogen atom or a methyl group, Y represents a substituted or unsubstituted alkenyl group having 1 to 5 carbon atoms, and n represents 0 or 1.
<4> The dental adhesive composition according to any one of <1> to <3>, wherein the acidic group in the (meth)acrylate compound (1) is a phosphoric acid group.
<5> The dental adhesive composition according to any one of <1> to <4>, in which the content of the (meth)acrylate compound (1) is 1 part by mass to 20 parts by mass with respect to 100 parts by mass of the sum of the polymerizable compounds contained in the dental adhesive composition.
<6> The dental adhesive composition according to any one of <1> to <5>, in which the content of the polymerizable compound (2) is 1 part by mass to 40 parts by mass with respect to 100 parts by mass of the sum of the polymerizable compounds contained in the dental adhesive composition.
<7> The dental adhesive composition according to any one of <1> to <6>, in which the total content of the (meth)acrylate compound (1) and the polymerizable compound (2) is 1% by mass to 40% by mass with respect to the total mass of the dental adhesive composition.
<8> The dental adhesive composition described in any one of <1> to <7>, further including a (meth)acrylate compound (3) having two or more (meth)acryloyloxy groups.
<9> The dental adhesive composition according to <8>, wherein the (meth)acrylate compound (3) contains at least one selected from the group consisting of a (meth)acrylate compound having two or more urethane bonds and a (meth)acrylate compound having two or more thiourethane bonds.
<10> The dental adhesive composition according to <8> or <9>, wherein the (meth)acrylate compound (3) contains a reaction product of a thiol compound having two or more thiol groups, an iso(thio)cyanate compound having two or more iso(thio)cyanate groups, and a (meth)acrylate compound (4) having a hydroxy group.
<11> The dental adhesive composition according to any one of <8 to <10>, in which the content of the (meth)acrylate compound (3) is 10 parts by mass to 95 parts by mass with respect to 100 parts by mass of the sum of the polymerizable compounds contained in the dental adhesive composition.
<12> The dental adhesive composition according to any one of <8> to <11>, in which the total content of the (meth)acrylate compound (1), the polymerizable compound (2), and the (meth)acrylate compound (3) is 30% by mass to 90% by mass with respect to the total mass of the dental adhesive composition.
<13> The dental adhesive composition according to any one of <1> to <12>, containing an organic solvent and water.
<14> The dental adhesive composition according to <13>, in which the total content of the organic solvent and the water is 20% by mass to 70% by mass with respect to the total mass of the dental adhesive composition.
<15> The dental adhesive composition described in any one of <1> to <14>, in which the content of the silane coupling agent is less than 0.10% by mass with respect to the total mass of the dental adhesive composition.
<16> The dental adhesive composition according to any one of <1> to <15>, further containing a polymerization initiator.
<17> The dental adhesive composition according to any one of <1> to <16>, which is a dental bonding material.
<18> A dental material including a cured product of the dental adhesive composition according to any one of <1> to <17>.

### Effects of Invention

According to one embodiment of the first embodiment, it is possible to provide a dental adhesive composition which is excellent in adhesiveness in a cured product obtained when cured and has good storage stability, and a dental material obtained from the dental adhesive composition.

According to one embodiment of the second embodiment, it is possible to provide a dental adhesive composition capable of improving adhesiveness to a tooth substance and a porcelain material in a cured product obtained when cured.

In addition, according to another embodiment of the second embodiment, it is possible to provide a dental material having excellent adhesiveness to a tooth substance and a porcelain material.

### DESCRIPTION OF EMBODIMENTS

In the present disclosure, a numerical range represented using "to" means a range including numerical values described before and after "to" as a lower limit value and an upper limit value.

In the present disclosure, when there are a plurality of substances corresponding to each component in the composition, the amount of each component in the composition means the total amount of the plurality of substances present in the composition unless otherwise specified.

In the numerical ranges described in stages in the present disclosure, the upper limit value or the lower limit value described in one numerical range may be replaced with the upper limit value or the lower limit value of the numerical range described in another stage. In addition, in the numerical range described in the present disclosure, the upper limit value or the lower limit value of the numerical range may be replaced with a value shown in examples.

In the present disclosure, "(meth)acryloyl" means acryloyl or methacryloyl, and "(meth)acrylate" means acrylate or methacrylate.

In the present disclosure, "iso(thio)cyanate " means isocyanate or isothiocyanate.

In the present disclosure, the "carboxylic acid (ester)" means a carboxylic acid or a carboxylic acid ester.

The dental adhesive composition of the present disclosure includes the dental adhesive compositions of the first and second embodiments described below.

### [First Embodiment]

### «Dental Adhesive Composition»

The dental adhesive composition of the first embodiment contains a (meth)acrylate compound (1) having an acidic group, and at least one polymerizable compound (2) selected from the group consisting of a cyclopolymerizable compound (2a) and a polymerizable compound (2b) having a cyclic structure and one polymerizable group.

By containing the (meth)acrylate compound (1) and the polymerizable compound (2), the dental adhesive composition of the first embodiment is excellent in adhesiveness in a cured product obtained when cured, and has good storage stability.

### <(Meth)acrylate Compound (1)>

The dental adhesive composition of the first embodiment contains a (meth)acrylate compound (1) having an acidic group.

The (meth)acrylate compound (1) has affinity with an adherend and has a decalcification effect on a tooth substance.

The (meth)acrylate compound (1) may be contained singly or in combination of two or more kinds thereof.

The (meth)acrylate compound (1) naturally has a (meth)acryloyloxy group. The number of (meth)acryloyloxy groups contained in the (meth)acrylate compound (1) may be one or two or more, but is preferably one or two.

Examples of the acidic group in the (meth)acrylate compound (1) include a phosphoric acid group, a pyrophosphoric acid group, a thiophosphoric acid group, a phosphonic acid group, a sulfonic acid group, and a carboxylic acid group.

Among the above, the acidic group in the (meth)acrylate compound (1) is preferably at least one selected from the group consisting of a phosphoric acid group and a carboxylic acid group, and more preferably a phosphoric acid group.

Examples of the (meth)acrylate compound (1) having a phosphoric acid group include (meth)acryloyloxyalkyl dihydrogen phosphates such as 10-(meth)acryloyloxydecyl dihydrogen phosphate (MDP), and the like; bis[2-(meth)acryloyloxyethyl] hydrogen phosphate, bis[4-(meth)acryloyloxybutyl] hydrogen phosphate, bis[6-(meth)acryloyloxyhexyl] hydrogen phosphate, bis[8-(meth)acryloyloxyoctyl] hydrogen phosphate, bis[9-(meth)acryloyloxynonyl] hydrogen phosphate, bis[10-(meth)acryloyloxy decyl] hydrogen phosphate, 1,3-di(meth)acryloyloxypropyl dihydrogen phosphate, 2-(meth)acryloyloxyethyl phenyl hydrogen phosphate, 2-(meth)acryloyloxyethyl-2-bromoethyl hydrogen phosphate, bis[2-(meth)acryloyloxy-(1-hydroxymethyl)ethyl] hydrogen phosphate, and acid chlorides, alkali metal salts, and ammonium salts thereof.

Examples of the (meth)acrylate compound (1) having a pyrophosphoric acid group include bis[2-(meth)acryloyloxyethyl] pyrophosphate, bis[4-(meth)acryloyloxybutyl] pyrophosphate, and acid chlorides, alkali metal salts, and ammonium salts thereof.

Examples of the (meth)acrylate compound (1) having a thiophosphoric acid group include 2-(meth)acryloyloxyethyl dihydrogen thiophosphate, 3-(meth)acryloyloxypropyl dihydrogen thiophosphate, and acid chlorides, alkali metal salts, and ammonium salts thereof.

Examples of the (meth)acrylate compound (1) having a phosphonic acid group include 2-(meth)acryloyloxyethyl phenylphosphonate, 5-(meth)acryloyloxypentyl-3-phosphonopropionate, and acid chlorides, alkali metal salts, and ammonium salts thereof.

Examples of the (meth)acrylate compound (1) having a sulfonic acid group include 2-(meth)acrylamide-2-methylpropane sulfonic acid, styrene sulfonic acid, and 2-sulfoethyl (meth)acrylate.

Examples of the (meth)acrylate compound (1) having a carboxylic acid group include a (meth)acrylate compound (1) having one carboxy group in the molecule and a (meth)acrylate compound (1) having a plurality of carboxy groups in the molecule.

Examples of the (meth)acrylate compound (1) having one carboxy group in the molecule include (meth)acrylic acid, N-(meth)acryloylglycine, N-(meth)acryloylaspartic acid, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, p-vinylbenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, 2-(meth)acryloyloxyethyl hydrogen succinate, 2-(meth)acryloyloxyethyl hydrogen phthalate, 2-(meth)acryloyloxyethyl hydrogen malate, and acid halides thereof.

Examples of the (meth)acrylate compound (1) having a plurality of carboxy groups in the molecule include 6-(meth)acryloyloxyhexane-1,1-dicarboxylic acid, 9-(meth)acryloyloxynonane-1,1-dicarboxylic acid, 10-(meth)acryloyloxydecane-1,1-dicarboxylic acid, 11-(meth)acryloyloxyundecane-1,1-dicarboxylic acid, 12-(meth)acryloyloxydecane-1,1-dicarboxylic acid, 13 -(meth)acryloyloxytridecane-1,1-dicarboxylic acid, 4-(meth)acryloyloxyethyl trimellitate, 4-(meth)acryloyloxyethyl trimellitate anhydride, 4-(meth)acryloyloxybutyl trimellitate, 4-(meth)acryloyloxyhexyl trimellitate, 4-(meth)acryloyloxydecyl trimellitate, 2-(meth)acryloyloxyethyl-3'-(meth)acryloyloxy-2'-(3,4-dicarboxybenzoyloxy)propyl succinate, and acid anhydrides or acid halides thereof.

Among the above, from the viewpoint of adhesive strength to an adherend, the (meth)acrylate compound (1) is preferably 10-(meth)acryloyloxydecyl dihydrogen phosphate (MDP), 1,3-di(meth)acryloyloxypropyl dihydrogen phosphate, 2-(meth)acryloyloxyethyl dihydrogen phosphate, 4-(meth)acryloyloxyethyl trimellitate anhydride, 4-(meth)acryloyloxyethyl trimellitate, 2-(meth)acrylamide-2-methylpropane sulfonic acid, or 11-(meth)acryloyloxyundecane-1,1-dicarboxylic acid.

The content of the (meth)acrylate compound (1) is preferably 1 part by mass to 20 parts by mass, more preferably 3 parts by mass to 18 parts by mass, and still more preferably 4 parts by mass to 15 parts by mass with respect to 100 parts by mass of the sum of the polymerizable compounds contained in the dental adhesive composition.

When the content of the (meth)acrylate compound (1) is 1 part by mass or more, it is easy to obtain high adhesiveness to various adherends.

When the content of the (meth)acrylate compound (1) is 20 parts by mass or less, it is easy to maintain a balance between polymerizability and adhesiveness.

The polymerizable compound contained in the dental adhesive composition of the first embodiment means the (meth)acrylate compound (1), the polymerizable compound (2), the (meth)acrylate compound (3) described later, and any polymerizable compound.

### <Polymerizable Compound (2)>

The dental adhesive composition of the first embodiment contains at least one polymerizable compound (2) selected from the group consisting of a cyclopolymerizable compound (2a) and a polymerizable compound (2b) having a cyclic structure and one polymerizable group.

### (Cyclopolymerizable Compound (2a))

The cyclopolymerizable compound (2a) is not particularly limited as long as it is a polymerizable compound that is polymerized with cyclization.

In the case of a polymerizable compound capable of performing cyclization polymerization, moderate fluidity is exhibited before curing since a cyclic structure is not formed, and mechanical strength can be increased after curing since a cyclic structure is formed.

The cyclopolymerizable compound (2a) contained in the dental adhesive composition of the first embodiment may be contained only singly or in combination of two or more kinds thereof.

Examples of the cyclopolymerizable compound (2a) include a 1,6-diene-2-carboxylic acid (ester) monomer, and a 1,5-diene-2-carboxylic acid (ester) monomer.

As the 1,6-diene-2-carboxylic acid (ester) monomer, a monomer represented by the following Formula (a) can be suitably used.

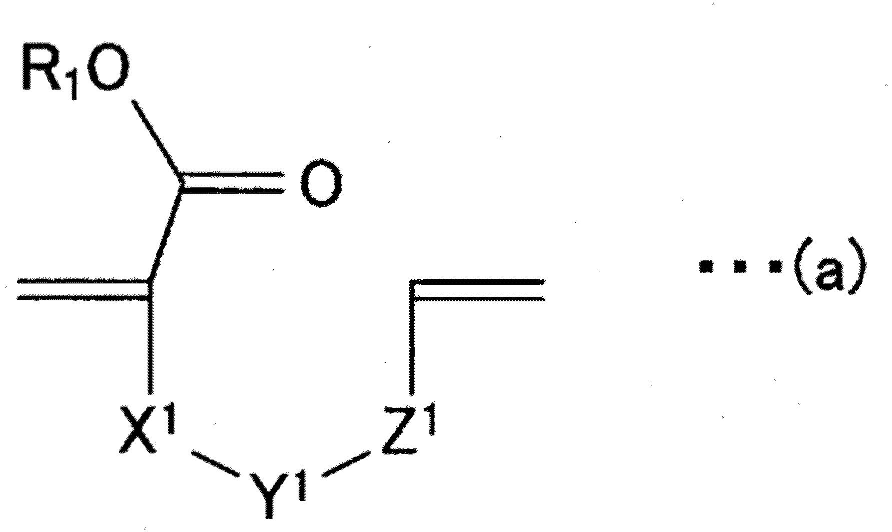

In Formula (a), R₁ represents a hydrogen atom or a substituted or unsubstituted hydrocarbon group having 1 to 20 carbon atoms.

In Formula (a), X¹, Y¹, and Z¹ are each independently an alkylene group, an oxygen atom, or an imino group. Here, at least one of X¹, Y¹, or Z¹ is an oxygen atom or an imino group.

Examples of the 1,6-diene-2-carboxylic acid (ester) monomer represented by Formula (a) include allyloxymethyl acrylic acid esters, and 2-(N-allylaminomethyl)acrylic acid esters.

Examples of the allyloxymethylacrylic acid esters include methyl 2-(allyloxymethyl)acrylate, ethyl 2-(allyloxymethyl)acrylate, butyl 2-(allyloxymethyl)acrylate, t-butyl 2-(allyloxymethyl)acrylate, cyclohexyl 2-(allyloxymethyl)acrylate, dicyclopentadienyl 2-(allyloxymethyl)acrylate, isobornyl 2-(allyloxymethyl)acrylate, adamantyl 2-(allyloxymethyl)acrylate, and benzyl 2-(allyloxymethyl)acrylate.

Examples of the 2-(N-allylaminomethyl)acrylic acid esters include methyl 2-(N-allyl-N-methylaminomethyl)acrylate, methyl 2-(N-allyl-N-ethylaminomethyl)acrylate, methyl 2-(N-allyl-N-t-butylaminomethyl)acrylate, methyl 2-(N-allyl-N-cyclohexylaminomethyl)acrylate, and methyl 2-(N-allyl-N-phenylaminomethyl)acrylate.

As the 1,6-diene-2-carboxylic acid (ester) monomer represented by Formula (a), allyloxymethylacrylic acid esters can be more preferably used.

As the allyloxymethylacrylic acid esters, at least one selected from the group consisting of methyl 2-(allyloxymethyl)acrylate, ethyl 2-(allyloxymethyl)acrylate, cyclohexyl 2-(allyloxymethyl)acrylate, and benzyl 2-(allyloxymethyl)acrylate can be further suitably used, and methyl 2-allyloxymethyl)acrylate (AOMA) can be particularly suitably used.

As the 1,5 diene-2-carboxylic acid (ester) monomer, a monomer having a structure represented by the following Formula (b) can be suitably used.

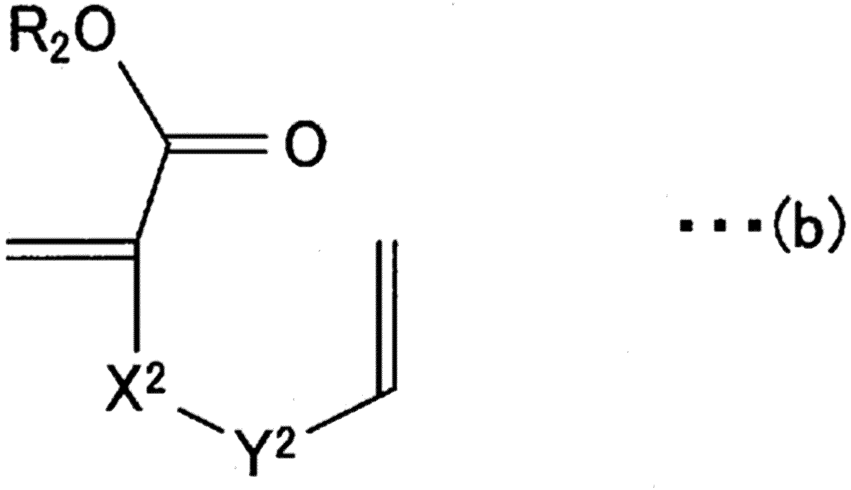

In Formula (b), R₂ represents a hydrogen atom or a substituted or unsubstituted hydrocarbon group having 1 to 20 carbon atoms.

In Formula (b), X² and Y² are each independently an alkylene group, an oxygen atom, or an imino group. Here, at least one of X² or Y² is an oxygen atom or an imino group.

In Formulae (a) and (b), examples of the substituent in the hydrocarbon group include a halogen atom.

When the dental adhesive composition of the first embodiment contains a 1,6 diene-2-carboxylic acid (ester) monomer and a 1,5 diene-2-carboxylic acid (ester) monomer, R₁ and R₂ in the above Formulas (a) and (b) may have the same structure or different structures.

Examples of the 1,5-diene-2-carboxylic acid (ester) monomer represented by Formula (b) include vinyloxymethyl acrylic acid esters, N-methyl-N-vinyl-2-(methoxycarbonyl)allylamines, and 2-(N-vinylaminomethyl)acrylic acid esters.

Specific examples of the vinyl oxymethyl acrylic acid esters include methyl α-vinyloxymethylacrylate, ethyl α-vinyloxymethylacrylate, butyl α-vinyloxymethylacrylate, t-butyl α-vinyloxymethylacrylate, cyclohexyl α-vinyloxymethylacrylate, dicyclopentadienyl α-vinyloxymethylacrylate, isobornyl α-vinyloxymethylacrylate, adamantyl α-vinyloxymethylacrylate, and benzyl α-vinyloxymethylacrylate.

Examples of the N-methyl-N-vinyl-2-(methoxycarbonyl)allylamines include N-methyl-N-vinyl-2-(methoxycarbonyl)allylamine, N-ethyl-N-vinyl-2-(methoxycarbonyl)allylamine, N-t-butyl-N-vinyl-2-(methoxycarbonyl)allylamine, N-cyclohexyl-N-vinyl-2-(methoxycarbonyl)allylamine, and N-phenyl-N-vinyl-2-(methoxycarbonyl)allylamine.

Examples of the 2-(N-vinylaminomethyl)acrylic acid esters include methyl 2-(N-vinyl-N-methylaminomethyl)acrylate, methyl 2-(N-vinyl-N-ethylaminomethyl)acrylate, methyl 2-(N-vinyl-N-t-butylaminomethyl)acrylate, methyl 2-(N-vinyl-N-cyclohexylaminomethyl)acrylate, and methyl 2-(N-vinyl-N-phenylaminomethyl)acrylate.

As the 1,5-diene-2-carboxylic acid (ester) monomer represented by Formula (b), in particular, at least one selected from the group consisting of methyl α-vinyloxymethylacrylate, ethyl α-vinyloxymethylacrylate, cyclohexyl α-vinyloxymethylacrylate, and benzyl α-vinyloxymethylacrylate can be more preferably used, and methyl α-vinyloxymethylacrylate can be still more preferably used.

Among the above, the cyclopolymerizable compound (2a) preferably contains a 1,6-diene-2-carboxylic acid (ester) monomer.

The cyclopolymerizable compound (2a) preferably contains an acrylic acid ester compound (2a-1) having one allyloxy group.

As the acrylic acid ester compound (2a-1), the specific examples exemplified in the allyloxymethylacrylic acid esters can be used.

As the cyclopolymerizable compound (2a), the acrylic acid ester compound (2a-1) is particularly preferably a compound represented by the following Formula (2a-1a).

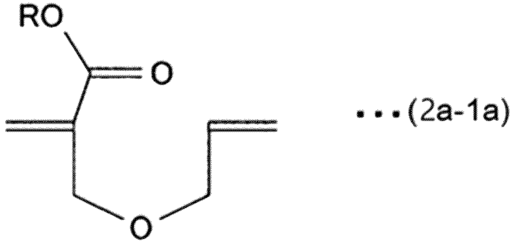

In Formula (2a-1a), R represents a hydrogen atom or a substituted or unsubstituted hydrocarbon group having 1 to 20 carbon atoms.

In Formula (2a-1a), examples of the substituent in the hydrocarbon group include a halogen atom.

### (Polymerizable Compound (2b))

The polymerizable compound (2b) has a cyclic structure and one polymerizable group.

Examples of the cyclic structure in the polymerizable compound (2b) include an alicyclic ring, an aromatic ring, and a heterocyclic ring.

The number of carbon atoms in the cyclic structure is preferably 3 to 12, more preferably 4 to 10, and still more preferably 5 to 8.

The polymerizable compound (2b) may have only one cyclic structure or two or more cyclic structures.

Examples of the polymerizable group include an alkenyl group, and a (meth)acryloyloxy group.

The polymerizable compound (2b) preferably contains a (meth)acrylate compound (2b-1) having a cyclic structure having 3 to 12 carbon atoms and one (meth)acryloyloxy group.

In addition, the (meth)acrylate compound (2b-1) is more preferably a compound represented by the following Formula (2b-1a).

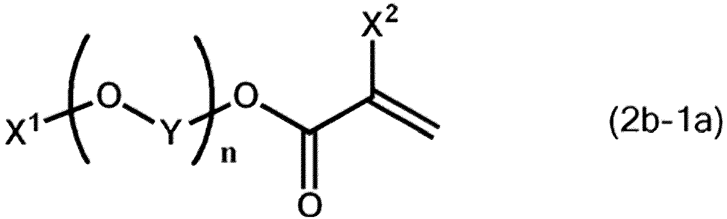

In Formula (2b-1a), X¹ represents an alicyclic, aromatic, or heterocyclic ring having 4 to 10 carbon atoms, or an alkyl group having 1 to 3 carbon atoms substituted with an alicyclic, aromatic, or heterocyclic ring having 4 to 10 carbon atoms, X² represents a hydrogen atom or a methyl group, Y represents a substituted or unsubstituted alkenyl group having 1 to 5 carbon atoms, and n represents 0 or 1.

X¹ is preferably an alicyclic ring, an aromatic ring, or a heterocyclic ring having 6 carbon atoms.

The "alicyclic ring" in X¹ includes both a substituted alicyclic ring and an unsubstituted (that is, unsubstituted) alicyclic ring. The "aromatic ring" in X¹ includes both a substituted aromatic ring and an unsubstituted aromatic ring. The "heterocyclic ring" in X¹ includes both a substituted heterocyclic ring and an unsubstituted heterocyclic ring.

The number of carbon atoms in an alicyclic ring, an aromatic ring, and a heterocyclic alicyclic ring in X¹ means the number of carbon atoms forming the ring. That is, the number of carbon atoms that do not form a ring (for example, carbon atoms of an alkyl group and the like) is not included in the number of carbon atoms of an alicyclic ring, an aromatic ring, and a heterocyclic alicyclic ring.

Examples of the substituent of an alicyclic ring, an aromatic ring, and a heterocyclic ring in X¹ include an alkyl group having 1 to 6 carbon atoms, a hydroxyalkyl group having 1 to 6 carbon atoms, and a hydroxy group.

Examples of the substituent in Y include a hydroxy group and the like, and a hydroxy group is preferable.

The number of carbon atoms in Y is preferably 2 to 4, and more preferably 2 or 3.

Examples of the polymerizable compound (2b) include compounds represented by the following Formulas (2b-1a-1) to (2b-1a-42). The polymerizable compound (2b) in the first embodiment is not limited thereto.

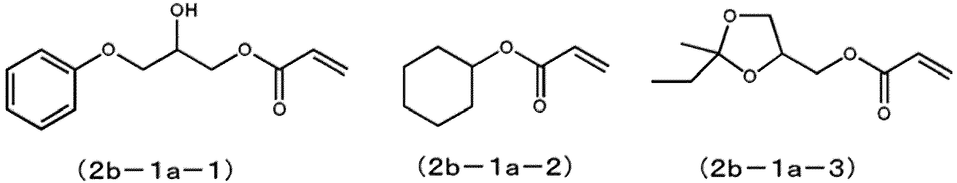

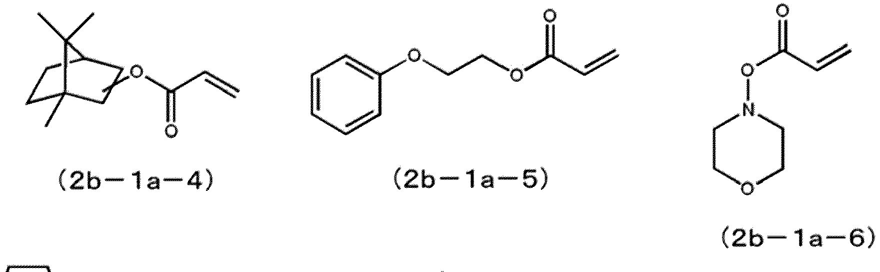

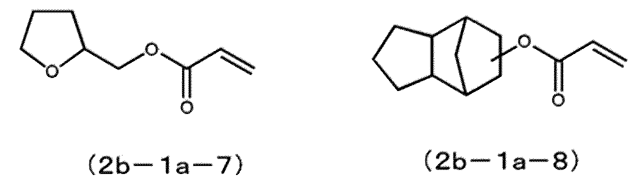

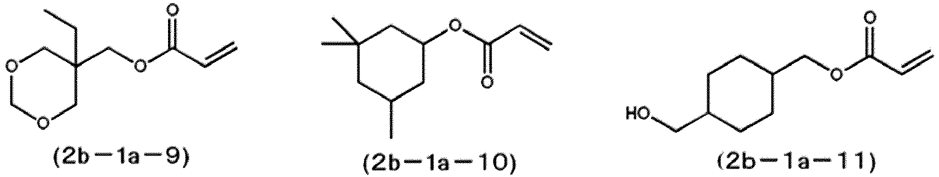

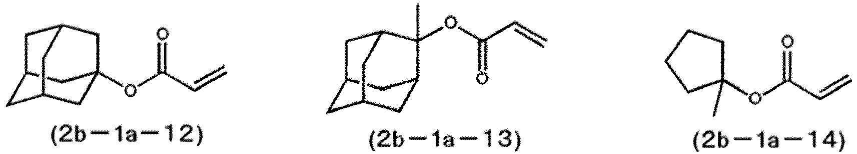

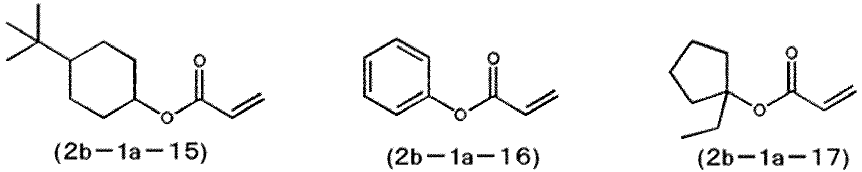

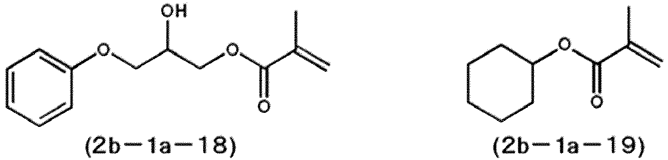

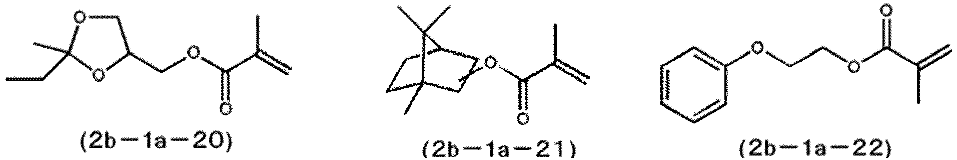

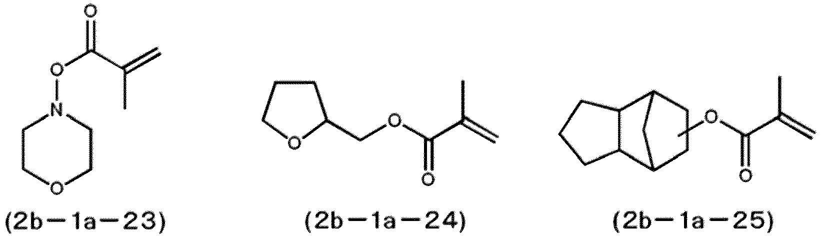

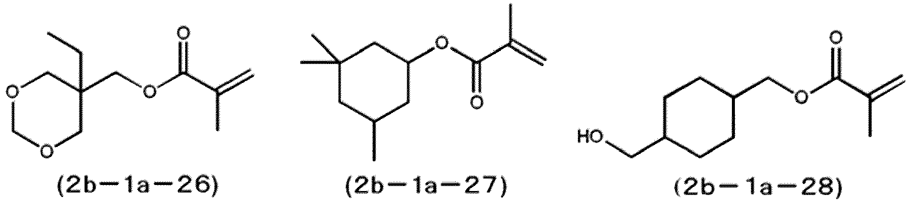

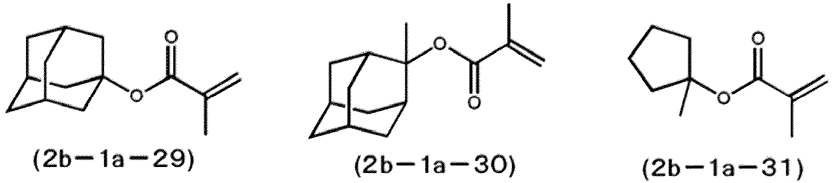

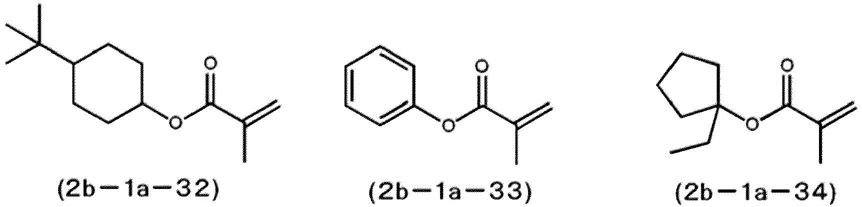

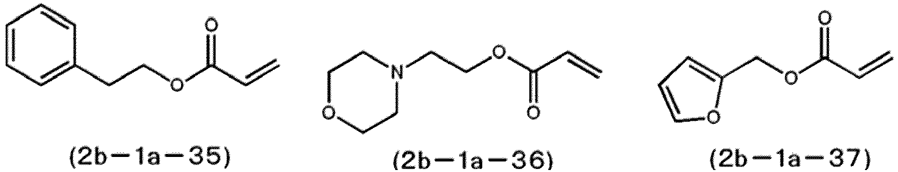

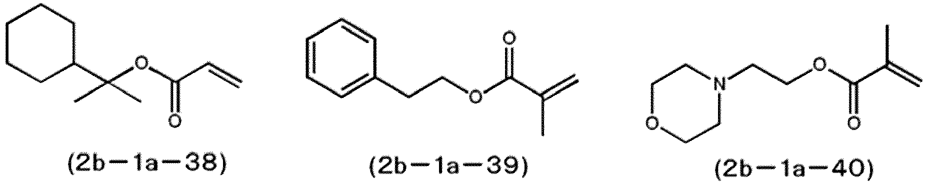

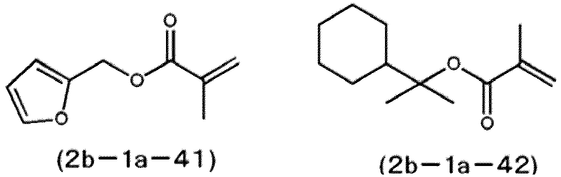

The polymerizable compound (2b) is preferably at least one selected from the group consisting of 2-hydroxy-3-phenoxypropyl (meth)acrylate, cyclohexyl (meth)acrylate, (2-methyl-2-ethyl-1,3-dioxolan-4-yl)methyl (meth)acrylate, isobornyl (meth)acrylate, phenoxyethyl (meth)acrylate, 4-(meth)acryloylmorpholine, tetrahydrofurfuryl (meth)acrylate, dicyclopentanyl (meth)acrylate, 5-ethyl-1,3-dioxan-5-ylmethyl (meth)acrylate, 3,3,5-trimethylcyclohexyl (meth)acrylate, 1,4cyclohexanedimethanol mono(meth)acrylate, 1-adamantyl (meth)acrylate, 2-methyl-2-adamantyl (meth)acrylate, 1-methylcyclopentyl (meth)acrylate, 4-tert-butylcyclohexyl (meth)acrylate, phenyl (meth)acrylate, 1-ethylcyclopentyl (meth)acrylate, 2-phenoxyethyl (meth)acrylate, 2-morpholinoethyl (meth)acrylate, furfuryl (meth)acrylate, and 2-cyclohexylpropan-2-yl (meth)acrylate.

It is preferable for the dental adhesive composition of the first embodiment that
the cyclopolymerizable compound (2a) contains an acrylic acid ester compound (2a-1) having one allyloxy group, and
the polymerizable compound (2b) contains a (meth)acrylate compound (2b-1) having a cyclic structure having 3 to 12 carbon atoms and one (meth)acryloyloxy group.

It is more preferable for the dental adhesive composition of the first embodiment that the acrylic acid ester compound (2a-1) contains a compound represented by Formula (2a-1a), and the (meth)acrylate compound (2b-1) contains a compound represented by Formula (2b-1a).

It is preferable for the dental adhesive composition of the first embodiment that the polymerizable compound (2) is at least one selected from the group consisting of a compound represented by the following Formula (2a-1a-1) and compounds represented by the above Formulas (2b-1a-1) to (2b-1a-42).

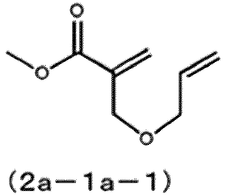

The content of the polymerizable compound (2) is preferably 1 part by mass to 40 parts by mass, more preferably 5 parts by mass to 30 parts by mass, and still more preferably 13 parts by mass to 23 parts by mass with respect to 100 parts by mass of the sum of the polymerizable compounds contained in the dental adhesive composition.

The total content of the (meth)acrylate compound (1) and the polymerizable compound (2) is preferably 1% by mass to 40% by mass, more preferably 3% by mass to 30% by mass, and still more preferably 5% by mass to 25% by mass with respect to the total mass of the dental adhesive composition.

### <Optional Polymerizable Compound>

The dental adhesive composition of the first embodiment may contain optional polymerizable compounds other than the (meth)acrylate compound (1) and the polymerizable compound (2) described above.

Examples of the optional polymerizable compound include the following (meth)acrylate compound (3).

### ((Meth)acrylate Compound (3))

The dental adhesive composition of the first embodiment may further contain a (meth)acrylate compound (3) having two or more (meth)acryloyloxy groups.

The (meth)acrylate compound (3) may have two to six (meth)acryloyloxy groups, two to five (meth)acryloyloxy groups, or two to four (meth)acryloyloxy groups.

The (meth)acrylate compound (3) is preferably a compound having no acidic group.

The (meth)acrylate compound (3) may be contained singly or in combination of two or more kinds thereof.

The (meth)acrylate compound (3) is a compound which is polymerized by a radical polymerization reaction caused by a polymerization initiator.

Examples of the (meth)acrylate compound (3) include bifunctional (meth)acrylate compounds and tri- or higher functional (meth)acrylate compounds.

Examples of the aromatic compound-based bifunctional (meth)acrylate compound include 2,2-bis((meth)acryloyloxyphenyl)propane, 2,2-bis[4-(3-(meth)acryloyloxy)-2-hydroxypropoxyphenyl]propane, 2,2-bis(4-(meth)acryloyloxyethoxyphenyl)propane, 2,2-bis(4-(meth)acryloyloxypolyethoxyphenyl)propane, and 2,2-bis(4-(meth)acryloyloxypolypropoxyphenyl)propane.

Among them, 2,2-bis[4-(3-(methacryloyloxy)-2-hydroxypropoxyphenyl)propane (commonly called "Bis-GMA") and 2,2-bis(4-methacryloyloxypolyethoxyphenyl)propane are preferable.

Examples of the aliphatic compound-based bifunctional (meth)acrylate compound include an aliphatic compound-based bifunctional (meth)acrylate having no urethane bond and no thiourethane bond, a (meth)acrylate compound having two or more urethane bonds, and a (meth)acrylate compound having two or more thiourethane bonds.

Examples of the aliphatic compound-based bifunctional (meth)acrylate having no urethane bond or thiourethane bond include erythritol di(meth)acrylate, sorbitol di(meth)acrylate, mannitol di(meth)acrylate, pentaerythritol di(meth)acrylate, dipentaerythritol di(meth)acrylate, glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,5-pentanediol di(meth)acrylate, 1,6-hexanediol di(meth)acrylate, and 1,10-decanediol di(meth)acrylate1,2-bis(3-methacryloyloxy-2-hydroxypropyloxy)ethane.

Among them, glycerol dimethacrylate, triethylene glycol dimethacrylate (TEGDMA), 1,6-hexanediol dimethacrylate (HexDMA), neopentyl glycol dimethacrylate (NPG), and 1,2-bis(3-methacryloyloxy-2-hydroxypropyloxy)ethane are preferable.

The (meth)acrylate compound having two or more urethane bonds can be produced, for example, as a reaction product of an iso(thio)cyanate compound having two or more iso(thio)cyanate groups and a (meth)acrylate compound (4) having a hydroxy group.

In the (meth)acrylate compound having two or more urethane bonds, the number of urethane bonds is preferably two.

As the (meth)acrylate compound having two or more urethane bonds, 2,2,4-trimethylhexamethylenebis(2-carbamoyloxyethyl)dimethacrylate (UDMA) is preferable.

The (meth)acrylate compound having two or more thiourethane bonds can be produced, for example, as a reaction product of a thiol compound having two or more thiol groups, an iso(thio)cyanate compound having two or more iso(thio)cyanate groups, and a (meth)acrylate compound (4) having a hydroxy group.

That is, the (meth)acrylate compound (3) preferably contains a reaction product of a thiol compound having two or more thiol groups, an iso(thio)cyanate compound having two or more iso(thio)cyanate groups, and a (meth)acrylate compound (4) having a hydroxy group.

In the (meth)acrylate compound having a thiourethane bond, the number of thiourethane bonds is preferably three or four.

In the present disclosure, the "(meth)acrylate compound having two or more urethane bonds" does not have two or more thiourethane bonds.

In the present disclosure, the "(meth)acrylate compound having two or more thiourethane bonds" may have two or more urethane bonds.

Examples of the tri- or higher functional (meth)acrylate compound include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol tri(meth)acrylate, dipentaerythritol tetra(meth)acrylate, dipentaerythritol penta(meth)acrylate, N,N-(2,2,4-trimethylhexamethylene)bis[2-(aminocarboxy)propane-1,3-diol] tetramethacrylate, and 1,7-diacryloyloxy-2,2,6,6-tetraacryloyloxymethyl-4-oxyheptane.

From the viewpoint of adhesiveness, the (meth)acrylate compound (3) preferably contains at least one selected from the group consisting of a (meth)acrylate compound having two or more urethane bonds and a (meth)acrylate compound having two or more thiourethane bonds.

### (Thiol Compound)

Examples of the thiol compound having two or more thiol groups include pentaerythritoltetrakis(3-mercaptopropionate), pentaerythritoltetrakis(2-mercaptoacetate), 4-mercaptomethyl-1,8-dimercapto-3,6-dithiaoctane, 5,7-dimercaptomethyl-1,11-dimercapto-3,6,9-trithiaundecane, 4,7-dimercaptomethyl-1,11-dimercapto-3,6,9-trithiaundecane, 4,8-dimercaptomethyl-1,11-dimercapto-3,6,9-trithiaundecane, 2,5-dimercaptomethyl-1,4-dithiane, 1,1,3,3-tetrakis(mercaptomethylthio)propane, 4,6-bis(mercaptomethylthio)-1,3-dithiane, 2-(2,2-bis(mercaptomethylthio)ethyl)-1,3-dithietane, 1,2-bis(mercaptomethyl)benzene, 1,3-bis(mercaptomethyl)benzene, 1,4-bis(mercaptomethyl)benzene, trimethylolpropanetris(3-mercaptopropionate), and tris-[(3-mercaptopropionyloxy)-ethyl]-isocyanurate.

Among the above,the thiol compound preferably contains at least one selected from the group consisting of 5,7-dimercaptomethyl-1,11-dimercapto-3,6,9-trithiaundecane, 4,7-dimercaptomethyl-1,11-dimercapto-3,6,9-trithiaundecane, 4,8-dimercaptomethyl-1,11-dimercapto-3,6,9-trithiaundecane, 4-mercaptomethyl-1,8-dimercapto-3,6-dithiaoctane, 1,1,3,3-tetrakis(mercaptomethylthio)propane, trimethylolpropanetris(3-mercaptopropionate), pentaerythritoltetrakis(2-mercaptoacetate), pentaerythritoltetrakis(3-mercaptopropionate), and tris- [(3-mercaptopropionyloxy)-ethyl]-isocyanurate.

The thiol compound may be used singly or in combination of two or more kinds thereof.

### (Iso(thio)cyanate Compound)

Examples of the iso(thio)cyanate compound having two or more iso(thio)cyanate groups include the following isocyanate compounds and isothiocyanate compounds.

Examples of the isocyanate compound include hexamethylene diisocyanate, 2,2,4-trimethylhexamethylene diisocyanate, 2,4,4-trimethylhexamethylene diisocyanate, pentamethylene diisocyanate, m-xylylene diisocyanate, 1,3-tetramethylxylylene diisocyanate, isophorone diisocyanate, bis(isocyanatemethyl)cyclohexane, bis(isocyanatecyclohexyl)methane, 2,5-bis(isocyanatemethyl)bicyclo-[2.2.1]-heptane, 2,6-bis(isocyanatemethyl)bicyclo-[2.2.1]-heptane, tolylene diisocyanate, phenylene diisocyanate, and 4,4 '-diphenylmethane diisocyanate.

The isocyanate compound may be used singly or in combination of two or more kinds thereof.

Examples of the isothiocyanate compound include aliphatic polyisothiocyanate compounds such as hexamethylene diisothiocyanate, lysine diisothiocyanate methyl ester, lysine triisothiocyanate, m-xylylene diisothiocyanate, bis(isothiocyanate methyl) sulfide, bis(isothiocyanate ethyl) sulfide, bis(isothiocyanate ethyl) disulfide, and the like; alicyclic polyisothiocyanate compounds such as isophorone diisothiocyanate, bis(isothiocyanatemethyl)cyclohexane, dicyclohexylmethane diisothiocyanate, cyclohexanediisothiocyanate, methylcyclohexanediisothiocyanate, 2,5-bis(isothiocyanatemethyl)bicyclo-[2.2.1]-heptane, 2,6-bis(isothiocyanatemethyl)bicyclo-[2.2.1]-heptane, 3,8-bis(isothiocyanatemethyl)tricyclodecane, 3,9-bis(isothiocyanatemethyl)tricyclodecane, 4,8-bis(isothiocyanatemethyl)tricyclodecane, 4,9-bis(isothiocyanatemethyl)tricyclodecane, and the like; aromatic polyisothiocyanate compounds such as tolylene diisothiocyanate, 4,4-diphenylmethane diisothiocyanate, diphenyl disulfide-4,4-diisothiocyanate, and the like; and sulfur-containing heterocyclic polyisothiocyanate compounds such as 2,5-diisothiocyanate thiophene, 2,5-bis(isothiocyanate methyl)thiophene, 2,5-isothiocyanate tetrahydrothiophene, 2,5-bis(isothiocyanate methyl)tetrahydrothiophene, 3,4-bis(isothiocyanate methyl)tetrahydrothiophene, 2,5-diisothiocyanate-1,4-dithiane, 2,5-bis(isothiocyanatemethyl)-1,4-dithiane, and 4,5-diisothiocyanate-1,3-dithiolane, 4,5-bis(isothiocyanatemethyl)-1,3-dithiolane, and the like.

The isothiocyanate compound may be used singly or in combination of two or more kinds thereof.

Among the above, the iso(thio)cyanate compound preferably contains at least one selected from the group consisting of hexamethylene diisocyanate, 2,2,4-trimethylhexamethylene diisocyanate, 2,4,4-trimethylhexamethylene diisocyanate, pentamethylene diisocyanate, m-xylylene diisocyanate, 1,3-tetramethylxylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, phenylene diisocyanate, and 4,4 '-diphenylmethane diisocyanate, and it is preferable to contain at least one selected from the group consisting of 2,2,4-trimethylhexamethylene diisocyanate and m-xylylene diisocyanate.

### ((Meth)acrylate Compound (4))

Examples of the (meth)acrylate compound (4) having a hydroxy group include 2-hydroxyethyl (meth)acrylate, 2-hydroxypropyl (meth)acrylate, 2-hydroxybutyl (meth)acrylate, 4-hydroxybutyl (meth)acrylate, 2-hydroxy-3 phenoxypropyl (meth)acrylate, and 1,4-cyclohexanedimethanol mono(meth)acrylate.

Among the above, the (meth)acrylate compound (4) preferably contains at least one selected from the group consisting of 2-hydroxyethyl (meth)acrylate and 2-hydroxypropyl (meth)acrylate.

The (meth)acrylate compound (4) may be used singly or in combination of two or more kinds thereof.

The content of the (meth)acrylate compound (3) is preferably 10 parts by mass to 95 parts by mass, more preferably 30 parts by mass to 97 parts by mass, and still more preferably 50 parts by mass to 95 parts by mass with respect to 100 parts by mass of the sum of the polymerizable compounds contained in the dental adhesive composition.

The total content of the (meth)acrylate compound having two or more urethane bonds and the compound having two or more thiourethane bonds is preferably 40 parts by mass to 95 parts by mass, more preferably 45 parts by mass to 90 parts by mass, and still more preferably 50 parts by mass to 80 parts by mass with respect to 100 parts by mass of the sum of the polymerizable compounds contained in the dental adhesive composition.

The content of the aliphatic compound-based bifunctional (meth)acrylate having no urethane bond and no thiourethane bond is preferably 1 part by mass to 50 parts by mass, more preferably 3 parts by mass to 40 parts by mass, and still more preferably 5 parts by mass to 30 parts by mass with respect to 100 parts by mass of the sum of the polymerizable compounds contained in the dental adhesive composition.

The dental adhesive composition of the first embodiment may contain optional polymerizable compounds other than the (meth)acrylate compound (1), the polymerizable compound (2), and the (meth)acrylate compound (3) described above.

Optional polymerizable compounds can be used without particular limitation, for example, for the purpose of imparting strength to a cured product, diluting a composition, improving the adhesiveness of a cured product, and the like.

Examples of the optional polymerizable compound include a (meth)acrylate compound (5) having one (meth)acryloyloxy group.

Examples of the (meth)acrylate compound (5) include the (meth)acrylate compound (4) having a hydroxy group (for example, 2-hydroxyethyl methacrylate (HEMA), etc.), decyl methacrylate (DEMA), and 3-methacryloyloxypropyltrimethoxysilane (γ-MPTS).

The dental adhesive composition of the first embodiment can be cured under appropriate conditions by the polymerization method of the polymerization initiator described above. For example, in the case of the dental adhesive composition of the first embodiment containing a photopolymerization initiator by visible light irradiation, the dental adhesive composition is processed into a predetermined shape, and then irradiated with visible light for a predetermined time using a known light irradiation device, whereby a desired cured product can be obtained. Conditions such as irradiation intensity, irradiation intensity, and the like can be appropriately changed according to the curability of the dental adhesive composition. In addition, the cured product cured by irradiation with light including visible light may be further subjected to heat treatment under appropriate conditions to improve the bending strength of the cured product.

### <Polymerization Initiator>

The dental adhesive composition of the first embodiment preferably further contains a polymerization initiator.

When the dental adhesive composition of the first embodiment contains a polymerization initiator, it has chemical polymerizability, thermal polymerizability, or photopolymerizability, and a cured product can be obtained more favorably.

As the polymerization initiator, a general polymerization initiator used in the dental field can be used, and usually, the polymerization initiator is selected in consideration of the polymerizability and polymerization conditions of a polymerizable compound such as the compound represented by Formula (1) contained in the dental adhesive composition, and the like.

When chemical polymerization is performed, the polymerization initiator is preferably, for example, a redox-based polymerization initiator obtained by combining an oxidizing agent and a reducing agent. When a redox-based polymerization initiator is used, the oxidizing agent and the reducing agent may be separately packaged and mixed immediately before use. The polymerization initiator is not particularly limited as long as it is a generally used known polymerization initiator, and can be used, and is usually selected in consideration of the polymerizability of the polymerizable compound and polymerization conditions thereof.

As the polymerization initiator, for example, redox polymerization initiators such as organic peroxide/aromatic amine types such as diacyl peroxides such as benzoyl peroxide, decanoyl peroxide, and the like, cumene hydroperoxide/thiourea types, ascorbic acid/copper salt types, organic peroxide/amine compound/sulfinic acid (or salt thereof) types, and the like can be used. As the polymerization initiator, trialkylboranes and partial oxides thereof such as tributylborane and a partial oxide thereof, and the like, 5-butylbarbituric acid, a 5-butyl-2-thiobarbituric acid-based catalyst, and the like are also suitably used.

When thermal polymerization is performed by heating, polymerization initiators such as a peroxide, an azo-based compound, and the like are preferable.

The peroxide is not particularly limited, and examples thereof include benzoyl peroxide, t-butyl hydroperoxide, and cumene hydroperoxide. The azo compound is not particularly limited, and examples thereof include azobisisobutyronitrile.

When photopolymerization by visible light irradiation is performed, photopolymerization catalysts are mentioned such as α-diketones such as camphorquinone, acetylbenzoyl, and the like; benzoyl alkyl ethers such as benzoyl ethyl ether, and the like, thioxanthone derivatives such as 2-chlorothioxanthone, methylthioxanthone, and the like, benzophenone derivatives such as benzophenone, p,p'-methoxybenzophenone, and the like; and acylphosphine oxides such as 2,4,6-trimethylbenzoyldiphenylphosphine oxide, 2,6-dimethoxybenzoyldiphenylphosphine oxide, and the like. Further, in addition to these polymerization initiators, it is preferable to use co-catalyst components such as amine compounds such as dimethylaminoethyl methacrylate, N,N-dimethyl-p-toluidine, ethyl N,N-dimethylbenzoate, and the like, aldehyde compounds such as citronellal, dimethylaminobenzaldehyde, and the like, and compounds having a thiol group such as 2-mercaptobenzoxazole, decanethiol, and the like.

The polymerization initiator may be used singly or in combination of two or more kinds thereof. The content of the polymerization initiator is preferably 0.01% by mass to 20% by mass, and more preferably 0.1% by mass to 5% by mass with respect to 100% by mass of the dental adhesive composition.

### <Filler>

The dental adhesive composition of the first embodiment may contain a filler. The filler is not particularly limited as long as it is a general filler used in the dental field, and can be used. The filler is generally roughly classified into an organic filler and an inorganic filler.

Examples of the organic filler include fine powders of polymethyl methacrylate, a methyl methacrylate-ethyl methacrylate copolymer, an ethylene-vinyl acetate copolymer, and a styrene-butadiene copolymer.

Examples of the inorganic filler include fine powders of various glasses (silicon dioxide is contained as a main component, and if necessary, an oxide of heavy metals, boron, aluminum, and the like is contained.), various ceramics, diatomaceous earth, kaolin, clay minerals (montmorillonite etc.), activated white clay, synthetic zeolite, mica, calcium fluoride, ytterbium fluoride, calcium phosphate, barium sulfate, zirconium dioxide, titanium dioxide, and hydroxyapatite. Specific examples of such an inorganic filler include barium borosilicate glass (shot 8235, shot GM 27884, shot G018-053, etc.), strontium boroaluminosilicate glass (shot G018-163, etc.), lanthanum glass (shot GM 31684, etc.), fluoroaluminosilicate glass (shot G018-117, etc.), and boroaluminosilicate glass containing zirconium, cesium (shot G018-307, etc.).

These fillers may be used singly or in combination of two or more kinds thereof. The content of the filler may be appropriately determined in consideration of the operability (consistency) of the dental adhesive composition (for example, the composite resin composition), the bending strength of the cured product thereof, and the like, and is preferably 10 parts by mass to 2000 parts by mass, more preferably 50 parts by mass to 1000 parts by mass, and still more preferably 100 parts by mass to 600 parts by mass, with respect to 100 parts by mass of all components other than the filler contained in the dental adhesive composition.

In addition, the dental adhesive composition of the first embodiment may be used for a purpose other than the composite resin composition.

When the dental adhesive composition of the first embodiment is used for a purpose other than the composite resin composition, the content of the filler is preferably less than 10 parts by mass, more preferably 5 parts by mass or less, and still more preferably 1 part by mass or less with respect to 100 parts by mass of all components other than the filler contained in the dental adhesive composition.

The dental adhesive composition of the first embodiment may not contain a filler. That is, the content of the filler may be 0 parts by mass with respect to 100 parts by mass of all components other than the filler contained in the dental adhesive composition.

### <Solvent>

The dental adhesive composition of the first embodiment preferably contains a solvent.

Examples of the solvent include an organic solvent and water.

The dental adhesive composition of the first embodiment preferably contains an organic solvent and water.

Conventionally, two types of dental bonding materials: aqueous bonding materials and non-aqueous bonding materials, have been used. The aqueous bonding material and the non-aqueous bonding material may be selectively used depending on an object to be bonded.

For example, when the object to be bonded is a porcelain material, a non-aqueous bonding material is often used. In particular, the storage stability of the non-aqueous bonding material tends to deteriorate over time. That is, it is considered that the conventional non-aqueous bonding material deteriorates storage stability due to intrusion of external water (for example, water vapor).

However, the dental adhesive composition of the first embodiment can contain a solvent, particularly water, and thus can maintain good storage stability even when used as an aqueous adhesive composition. In addition, good adhesiveness to a porcelain material is exhibited.

That is, the dental adhesive composition of the first embodiment can maintain good storage stability even if external water (for example, water vapor) intrudes.

As the organic solvent, ethanol, isopropyl alcohol, acetone, and the like can be used.

Examples of the water include distilled water and ultrapure water.

In the dental adhesive composition of the first embodiment, the content of water is preferably 1% by mass to 25% by mass, more preferably 5% by mass to 20% by mass, and still more preferably 10% by mass to 20% by mass with respect to the total mass of the dental adhesive composition.

In the dental adhesive composition of the first embodiment, the content of the organic solvent is preferably 10% by mass to 40% by mass, and more preferably 20% by mass to 30% by mass with respect to the total mass of the dental adhesive composition.

In the dental adhesive composition of the first embodiment, the total content of the organic solvent and water is preferably 20% by mass to 70% by mass, more preferably 30% by mass to 50% by mass, and still more preferably 35% by mass to 45% by mass with respect to the total mass of the dental adhesive composition.

### <Other Components>

The dental adhesive composition of the first embodiment may appropriately contain components other than the monomer composition of the first embodiment and the polymerization initiator according to the purpose.

The component that may be contained in the dental adhesive composition of the first embodiment is not particularly limited as long as it is a known component, and for example, a polymerization inhibitor may be contained in order to improve storage stability. In order to adjust the color tone, color matters such as known pigments, dyes, and the like may be contained. Furthermore, in order to improve the strength of a cured product, a reinforcing material such as a known fiber, and the like may be included. In addition, the dental adhesive composition of the first embodiment may contain additives such as a bactericidal agent, a disinfectant, a stabilizer, a preservative, and the like as necessary as long as the effects of the first embodiment are exhibited.

The dental adhesive composition of the first embodiment may or may not contain a silane coupling agent.

The content of the silane coupling agent is preferably less than 0.10% by mass, and more preferably less than 0.05% by mass (including a case where the content is 0% by mass) with respect to the total mass of the dental adhesive composition.

When the dental adhesive composition of the first embodiment contains a plurality of kinds of silane coupling agents, some of the silane coupling agents may be within the above content range, and all the silane coupling agents may be within the above content range.

In the present disclosure, the silane coupling agent refers to an organosilicon compound having a hydrolyzable group (for example, an alkoxy group or the like) capable of chemically bonding to an inorganic material by generating a hydroxy group by hydrolysis, and a reactive functional group (for example, a (meth)acryloyl group, an amino group, a vinyl group, an epoxy group, a mercapto group, etc.) capable of chemically bonding to an organic material.

### [Dental Material]

The dental material of the first embodiment contains a cured product of the dental adhesive composition of the first embodiment. The curing conditions of the dental adhesive composition may be appropriately determined according to the composition of the dental adhesive composition, the application of the dental material, and the like.

The cured product of the dental adhesive composition of the first embodiment obtained as described above can be suitably used as a dental material.

The method for using the dental adhesive composition of the first embodiment is not particularly limited as long as it is generally known as a method for using a dental material.

For example, when the dental adhesive composition of the first embodiment is used as a bonding material, the cavity in the oral cavity may be cleansed, the dental adhesive composition of the first embodiment may be filled, a tooth crown material such as a composite resin and the like may be filled, and photocuring may be performed using a known light irradiation device. As a result, the tooth crown material and a tooth substance (for example, enamel, dentin, etc.) can be bonded well.

The dental adhesive composition and the dental material of the first embodiment can be preferably used as, for example, a dental adhesive material such as a dental adhesive resin cement, a fissure blocking material, a bonding material, an orthodontic adhesive material, and the like.

Among the above, the dental adhesive composition and the dental material of the first embodiment are suitable for a bonding material. That is, the dental adhesive composition and the dental material of the first embodiment are preferably dental bonding materials.

The first embodiment includes the following aspects.
<1> A dental adhesive composition containing: a (meth)acrylate compound (1) having an acidic group; and at least one polymerizable compound (2) selected from the group consisting of a cyclopolymerizable compound (2a) and a polymerizable compound (2b) having a cyclic structure and one polymerizable group.
<2> The dental adhesive composition according to <1>, in which the cyclopolymerizable compound (2a) includes an acrylic acid ester compound (2a-1) having one allyloxy group, and the polymerizable compound (2b) includes a (meth)acrylate compound (2b-1) having a cyclic structure having 3 to 12 carbon atoms and one (meth)acryloyloxy group.
<3> The dental adhesive composition according to <1> or <2>, in which the polymerizable compound (2) is at least one selected from the group consisting of a compound represented by the following Formula (2a-1a) and a compound represented by the following Formula (2b-1a).

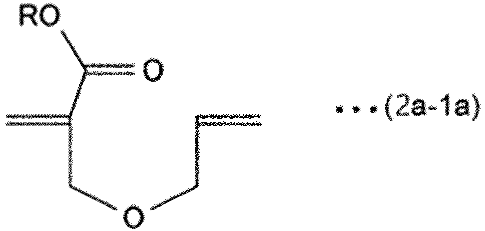

In Formula (2a-1a), R represents a hydrogen atom or a substituted or unsubstituted hydrocarbon group having 1 to 20 carbon atoms.

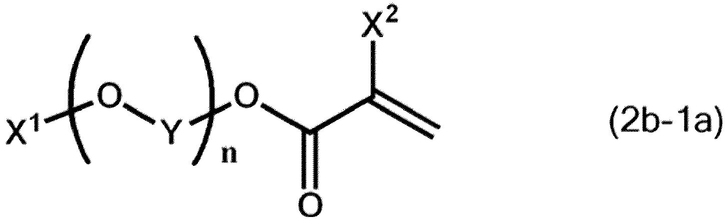

In Formula (2b-1a), X¹ represents an alicyclic, aromatic, or heterocyclic ring having 4 to 10 carbon atoms, or an alkyl group having 1 to 3 carbon atoms substituted with an alicyclic, aromatic, or heterocyclic ring having 4 to 10 carbon atoms, X² represents a hydrogen atom or a methyl group, Y represents a substituted or unsubstituted alkenyl group having 1 to 5 carbon atoms, and n represents 0 or 1.
<4> The dental adhesive composition according to any one of <1> to <3>, wherein the acidic group in the (meth)acrylate compound (1) is a phosphoric acid group.
<5> The dental adhesive composition according to any one of <1> to <4>, in which the content of the (meth)acrylate compound (1) is 1 part by mass to 20 parts by mass with respect to 100 parts by mass of the sum of the polymerizable compounds contained in the dental adhesive composition.
<6> The dental adhesive composition according to any one of <1> to <5>, in which the content of the polymerizable compound (2) is 1 part by mass to 40 parts by mass with respect to 100 parts by mass of the sum of the polymerizable compounds contained in the dental adhesive composition.
<7> The dental adhesive composition according to any one of <1> to <6>, in which the total content of the (meth)acrylate compound (1) and the polymerizable compound (2) is 1% by mass to 40% by mass with respect to the total mass of the dental adhesive composition.
<8> The dental adhesive composition according to any one of <1> to <7>, containing an organic solvent and water.
<9> The dental adhesive composition according to <8>, in which the total content of the organic solvent and the water is 20% by mass to 70% by mass with respect to the total mass of the dental adhesive composition.
<10> The dental adhesive composition according to any one of <1> to <9>, further containing a polymerization initiator.
<11> The dental adhesive composition according to any one of <1> to <10>, which is a dental bonding material.
<12> A dental material containing a cured product of the dental adhesive composition according to any one of <1> to <11>.

### [Second Embodiment]

The dental adhesive composition of the second embodiment is the dental adhesive composition of the first embodiment further containing a (meth)acrylate compound (3) having two or more (meth)acryloyloxy groups.

### «Dental Adhesive Composition»

The dental adhesive composition of the second embodiment contains a (meth)acrylate compound (1) having an acidic group, at least one polymerizable compound (2) selected from the group consisting of a cyclopolymerizable compound (2a) and a polymerizable compound (2b) having a cyclic structure and one polymerizable group, and a (meth)acrylate compound (3) having two or more (meth)acryloyloxy groups.

The dental adhesive composition according to the second embodiment contains the (meth)acrylate compound (1), the polymerizable compound (2), and the (meth)acrylate compound (3), thereby making it possible to improve the adhesiveness of a cured product obtained when cured to a tooth substance and a porcelain material.

### <(Meth)acrylate Compound (1)>

The dental adhesive composition of the second embodiment contains a (meth)acrylate compound (1) having an acidic group.

Details of specific examples, preferred specific examples, preferred contents and the like of the (meth)acrylate compound (1) in the second embodiment are the same as the details of specific examples, preferred specific examples, preferred contents and the like described in the section of the (meth)acrylate compound (1) in the first embodiment.

The polymerizable compound contained in the dental adhesive composition of the second embodiment means the (meth)acrylate compound (1), the (meth)acrylate compound (3), the polymerizable compound (2), and an optional polymerizable compound.

### <Polymerizable Compound (2)>

The dental adhesive composition of the second embodiment contains at least one polymerizable compound (2) selected from the group consisting of a cyclopolymerizable compound (2a) and a polymerizable compound (2b) having a cyclic structure and one polymerizable group.

Details of specific examples, preferred specific examples, and the like of the polymerizable compound (2) in the second embodiment are the same as the details of specific examples, preferred specific examples, and the like described in the section of the polymerizable compound (2) in the first embodiment.

The details of the preferred range and the like of the total content of the (meth)acrylate compound (1) and the polymerizable compound (2) in the second embodiment are the same as the details of the preferred range and the like of the total content of the (meth)acrylate compound (1) and the polymerizable compound (2) in the first embodiment.

The content of the polymerizable compound (2) in the second embodiment is preferably 1 part by mass to 40 parts by mass, more preferably 2 parts by mass to 30 parts by mass, and still more preferably 3 parts by mass to 20 parts by mass with respect to 100 parts by mass of the sum of the polymerizable compounds contained in the dental adhesive composition.

### <(Meth)acrylate Compound (3)>

The dental adhesive composition of the second embodiment contains a (meth)acrylate compound (3) having two or more (meth)acryloyloxy groups.

Details of specific examples, preferred specific examples, preferred contents and the like of the (meth)acrylate compound (3) in the second embodiment are the same as the details of specific examples, preferred specific examples, preferred contents and the like described in the section of the (meth)acrylate compound (3) in the first embodiment.

The (meth)acrylate compound (3) in the second embodiment preferably contains at least one selected from the group consisting of a (meth)acrylate compound having two or more urethane bonds and a (meth)acrylate compound having two or more thiourethane bonds, from the viewpoint of adhesiveness to a tooth substance and a porcelain material.

The total content of the (meth)acrylate compound (1), the polymerizable compound (2), and the (meth)acrylate compound (3) is preferably 30% by mass to 90% by mass, more preferably 40% by mass to 80% by mass, and still more preferably 45% by mass to 70% by mass with respect to the total mass of the dental adhesive composition.

### <Polymerization Initiator>

The dental adhesive composition of the second embodiment preferably further contains a polymerization initiator.

Details of specific examples, preferred specific examples, preferred contents, and the like of the polymerization initiator in the second embodiment are the same as the details of specific examples, preferred specific examples, preferred contents, and the like described in the section of the polymerization initiator in the first embodiment.

### <Filler>

The dental adhesive composition of the second embodiment may contain a filler.

Details of specific examples, preferred specific examples, preferred contents, and the like of the filler in the second embodiment are the same as the details of specific examples, preferred specific examples, preferred contents, and the like described in the section of the filler in the first embodiment.

### <Solvent>

The dental adhesive composition of the second embodiment preferably contains a solvent.

Examples of the solvent include an organic solvent and water.

The dental adhesive composition of the second embodiment preferably contains an organic solvent and water.

In the dental adhesive composition of the second embodiment, the total content of the organic solvent and water is preferably 20% by mass to 70% by mass, more preferably 30% by mass to 50% by mass, and still more preferably 35% by mass to 45% by mass with respect to the total mass of the dental adhesive composition.

Details of specific examples, preferred specific examples, preferred contents, and the like of the solvent in the second embodiment are the same as the details of specific examples, preferred specific examples, preferred content, and the like described in the section of the solvent in the first embodiment.

### <Other Components>

The dental adhesive composition of the second embodiment may appropriately contain components other than the monomer composition of the second embodiment and the polymerization initiator according to the purpose.

Details of specific examples, preferred specific examples, and the like of other components in the second embodiment are the same as the details of specific examples, preferred specific examples, and the like described in the section of other components in the first embodiment.

### (Optional Polymerizable Compound)

The dental adhesive composition of the second embodiment may contain optional polymerizable compounds other than the (meth)acrylate compound (1), the (meth)acrylate compound (3), and the polymerizable compound (2) described above.

The optional polymerizable compound can be used without particular limitation for the purpose of imparting strength to a cured product, diluting a composition, improving the adhesiveness of a cured product, and the like.

Details of specific examples, preferred specific examples, and the like of the optional polymerizable compound in the second embodiment are the same as the details of specific examples, preferred specific examples, and the like described in the section of the optional polymerizable compound in the first embodiment.

The dental adhesive composition of the second embodiment may or may not contain a silane coupling agent.

The details of the specific content, preferred content, and the like of the silane coupling agent in the second embodiment are the same as the details of the specific content, preferred content, and the like of the silane coupling agent in the first embodiment.

[Dental Material] The dental material of the second embodiment includes a cured product of the dental adhesive composition of the second embodiment. The curing conditions of the dental adhesive composition may be appropriately determined according to the composition of the dental adhesive composition, the application of the dental material, and the like.

The details of the specific method of use, the preferred method of use, the preferred application, and the like of the dental adhesive composition and the dental material in the second embodiment are the same as the details of the specific method of use, the preferred method of use, the preferred application, and the like of the dental adhesive composition and the dental material in the first embodiment.

The second embodiment includes the following aspects.
<1> A dental adhesive composition containing: a (meth)acrylate compound (1) having an acidic group; at least one polymerizable compound (2) selected from the group consisting of a cyclopolymerizable compound (2a) and a polymerizable compound (2b) having a cyclic structure and one polymerizable group; and a (meth)acrylate compound (3) having two or more (meth)acryloyloxy groups.
<2> The dental adhesive composition according to <1>, in which the cyclopolymerizable compound (2a) includes an acrylic acid ester compound (2a-1) having one allyloxy group, and the polymerizable compound (2b) includes a (meth)acrylate compound (2b-1) having a cyclic structure having 53 to 12 carbon atoms and one (meth)acryloyloxy group.
<3> The dental adhesive composition according to <1> or <2>, in which the polymerizable compound (2) is at least one selected from the group consisting of a compound represented by the following Formula (2a-1a) and a compound represented by the following Formula (2b-1a).

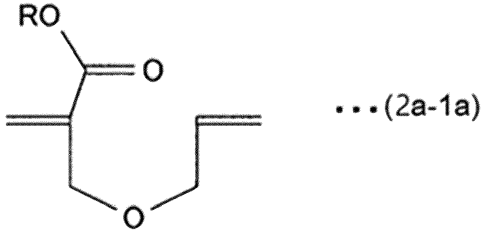

In Formula (2a-1a), R represents a hydrogen atom or a substituted or unsubstituted hydrocarbon group having 1 to 20 carbon atoms.

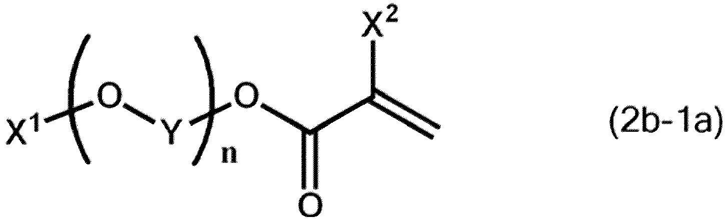

In Formula (2b-1a), X¹ represents an alicyclic, aromatic, or heterocyclic ring having 4 to 10 carbon atoms, or an alkyl group having 1 to 3 carbon atoms substituted with an alicyclic, aromatic, or heterocyclic ring having 4 to 10 carbon atoms, X² represents a hydrogen atom or a methyl group, Y represents a substituted or unsubstituted alkenyl group having 1 to 5 carbon atoms, and n represents 0 or 1.
<4> The dental adhesive composition according to any one of <1> to <3>, wherein the acidic group in the (meth)acrylate compound (1) is a phosphoric acid group.
<5> The dental adhesive composition according to any one of <1> to <4>, wherein the (meth)acrylate compound (3) contains at least one selected from the group consisting of a (meth)acrylate compound having two or more urethane bonds and a (meth)acrylate compound having two or more thiourethane bonds.
<6> The dental adhesive composition according to any one of <1> to <5>, wherein the (meth)acrylate compound (3) contains a reaction product of a thiol compound having two or more thiol groups, an iso(thio)cyanate compound having two or more iso(thio)cyanate groups, and a (meth)acrylate compound (4) having a hydroxy group.
<7> The dental adhesive composition according to any one of <1> to <6>, in which the content of the (meth)acrylate compound (1) is 1 part by mass to 20 parts by mass with respect to 100 parts by mass of the sum of the polymerizable compounds contained in the dental adhesive composition.
<8> The dental adhesive composition according to any one of <1> to <7>, in which the content of the (meth)acrylate compound (3) is 10 parts by mass to 95 parts by mass with respect to 100 parts by mass of the sum of the polymerizable compounds contained in the dental adhesive composition.
<9> The dental adhesive composition according to any one of <1> to <8>, in which the content of the polymerizable compound (2) is 1 part by mass to 40 parts by mass with respect to 100 parts by mass of the sum of the polymerizable compounds contained in the dental adhesive composition.
<10> The dental adhesive composition according to any one of <1> to <9>, in which the total content of the (meth)acrylate compound (1), the polymerizable compound (2), and the (meth)acrylate compound (3) is 30% by mass to 90% by mass with respect to the total mass of the dental adhesive composition.
<11> The dental adhesive composition according to any one of <1> to <10>, containing an organic solvent and water.
<12> The dental adhesive composition according to any one of <1> to <11>, in which the total content of the organic solvent and the water is 20% by mass to 70% by mass with respect to the total mass of the dental adhesive composition.
<13> The dental adhesive composition according to any one of <1> to <12>, further containing a polymerization initiator.
<14> The dental adhesive composition according to any one of <1> to <13>, which is a dental bonding material.
<15> A dental material containing a cured product of the dental adhesive composition according to any one of <1> to <14>.

### [Examples]

Hereinafter, examples of the first embodiment will be described, but the first embodiment is not limited by the following examples. Abbreviations of compounds used in examples of the first embodiment are shown below.

The abbreviations of compounds used in the present examples are shown below.
3G: triethylene glycol dimethacrylate
HEMA: 2-hydroxyethyl methacrylate
TMHDI: mixture of 2,2,4-trimethylhexamethylene diisocyanate and 2,4,4-trimethylhexamethylene diisocyanate
DBTDL: dibutyltin dilaurate
BHT: dibutylhydroxytoluene
UDMA: urethane dimethacrylate produced by a production method described below
AOMA: methyl 2-(allyloxymethyl)acrylate
CHA: cyclohexyl acrylate
HPPA: 1-hydroxy-3-phenylpropyl acrylate
PEA: 2-phenoxyethyl acrylate
MDP: 10-(phosphonooxy)decyl methacrylate
PEMA: 2-(phosphonooxy)ethyl methacrylate
4-MET: 4-methacryloxyethyl trimellitic acid
DEMA: decyl methacrylate
γ-MPTS: 3-methacryloyloxypropyltrimethoxysilane
CQ: camphorquinone
DMBE: 2-butoxyethyl 4-(dimethylamino)benzoate
EtOH: ethanol
DW: distilled water

### [Production Example 1: Production of urethane dimethacrylate (UDMA)]

Into a sufficiently dried 100 mL four-necked flask equipped with a stirring blade and a thermometer, 0.05 parts by mass of DBTDL, 0.025 parts by mass of BHT, and 22.34 parts by mass of TMHDI were charged and dissolved to form a homogeneous solution, and then this solution was heated to 80°C, and further 27.66 parts by mass of HEMA was added dropwise over 1 hour. Since the internal temperature increased due to the reaction heat during the dropwise addition, the amount of dropwise addition was controlled so as to be 90°C or lower. After the whole amount of HEMA was added dropwise, the reaction was performed for 3 hours while the reaction temperature was maintained at 90°C. At this time, the progress of the reaction was followed by HPLC analysis to confirm the end point of the reaction. The product was discharged from the reactor to obtain 50 g of urethane dimethacrylate.

### [Method of Adhesive Strength Test]

A method of an adhesive strength test in examples and comparative examples of the first embodiment will be described below.

### (Preparation of Test Piece for Adhesive Strength Test)

A porcelain disk-shaped flat plate (manufactured by VITA Corporation) was placed in a plastic cylindrical container having a diameter of 25 mm and a depth of 25 mm, and embedded in an acrylic resin. This surface was wet-polished using the emery paper of # 120 and # 400 to prepare a porcelain adherend.

Next, the flat surface was dried by blowing compressed air for about 1 second, and then the prepared composition was applied to the flat surface of the porcelain material, and weak blown compressed air was blown. The surface was irradiated with light for 20 seconds using a visible light irradiation device (Translux 2Wave, manufactured by Heraeus Kulzer Japan Co., Ltd.). Furthermore, a plastic mold having a diameter of 2.38 mm (manufactured by ULTRADENT PRODUCTS, INC.) was placed thereon, filled with a dental composite resin (Venus Diamond, manufactured by Heraeus Kulzer Japan Co., Ltd.), and the resin was irradiated with light for 20 seconds using a visible light irradiation device to be cured. Thereafter, the mold was removed to prepare an adhesive sample for a porcelain material.

### [Measuring Method in Adhesive Strength Test]

Before storing the sample and after storing the sample in warm water at 37°C for 24 hours, the following adhesive strength test was performed and used as an index of storage stability of the sample.

Using a general-purpose tester (Precision universal material testing machine 210 X, manufactured by INTESCO Co., Ltd.), various adhesion samples were subjected to a shear load at a crosshead speed of 1.0 mm/min in a state of being parallel and in contact with the surface. Then, the shear adhesive strength was determined from the shear load when the composition formed in a columnar shape on the sample surface was separated from the surface.

### Example 1

2.36 parts by mass of UDMA, 2.36 parts by mass of 3G, 0.29 parts by mass of MDP, 0.90 parts by mass of AOMA, 0.03 parts by mass of CQ, and 0.06 parts by mass of DMBE were put in a container, and the mixture was stirred at 50°C until it became uniform. Subsequently, 4 g of an aqueous solution adjusted to EtOH : DW = 3 : 2 was added, and the mixture was stirred at normal temperature until the mixture became uniform, thereby obtaining a monomer composition (1). An adhesive strength test was performed according to the methods described in (Preparation of Test Piece for Adhesive Strength Test) and (Curved Adhesive Strength Test) from the obtained monomer composition (1). The results are shown in Table 1.

### [Examples 2 to 6 and Comparative Examples 1 to 4]

A monomer composition (1) was prepared in the same manner as in Example 1 except that the composition of the monomer composition (1) was changed to the composition shown in Table 1, and an adhesive strength test was performed. The results are similarly shown in Table 1.

**[Table 1]**

| | | | Example 1 | Example 2 | Example 3 | Example 4 | Example 5 | Example 6 | Comparative Example 1 | Comparative Example 2 | Comparative Example 3 | Comparative Example 4 |
|---|---|---|---|---|---|---|---|---|---|---|---|---|
| Compound (1) | | MDP [parts by mass] | 0.29 | 0.29 | 0.29 | 0.29 | - | - | 0.29 | - | - | 0.29 |
| | | PEMA [parts by mass] | - | - | - | - | 0.29 | - | - | - | - | - |
| | | 4-MET [parts by mass] | - | - | - | - | - | 0.29 | - | - | - | - |
| Compound (2a) | | AOMA [parts by mass] | 0.90 | - | - | - | 0.90 | 0.90 | - | 1.19 | 0.90 | - |
| Compound (2b) | | CHA [parts by mass] | - | 0.90 | - | - | - | - | - | - | - | - |
| | | HPPA [parts by mass] | - | - | 0.9 | - | - | - | - | - | - | - |
| | | PEA [parts by mass] | - | - | - | 0.9 | - | - | - | - | - | - |
| Other polymerizable compounds | Compound (3) | UDMA [parts by mass] | 2.36 | 2.36 | 2.36 | 2.36 | 2.36 | 2.36 | 2.36 | 2.36 | 2.36 | 2.36 |
| | | 3G [parts by mass] | 2.36 | 2.36 | 2.36 | 2.36 | 2.36 | 2.36 | 2.36 | 2.36 | 2.36 | 2.36 |
| | Compound (5) | DEMA [parts by mass] | - | - | - | - | - | - | - | - | 0.29 | - |
| | | γ-MPTS [parts by mass] | - | - | - | - | - | - | 0.90 | - | - | - |
| Sum of polymerizable compounds [parts by mass] | | | 5.91 | 5.91 | 5.91 | 5.91 | 5.91 | 5.91 | 5.91 | 5.91 | 5.91 | 5.01 |
| Polymerization initiator | | CQ [parts by mass] | 0.03 | 0.03 | 0.03 | 0.03 | 0.03 | 0.03 | 0.03 | 0.03 | 0.03 | 0.03 |
| | | DMBE [parts by mass] | 0.06 | 0.06 | 0.06 | 0.06 | 0.06 | 0.06 | 0.06 | 0.06 | 0.06 | 0.06 |
| Aqueous diluting agent | | EtOH [parts by mass] | 2.4 | 2.4 | 2.4 | 2.4 | 2.4 | 2.4 | 2.4 | 2.4 | 2.4 | 2.4 |
| | | DW [parts by mass] | 1.6 | 1.6 | 1.6 | 1.6 | 1.6 | 1.6 | 1.6 | 1.6 | 1.6 | 1.6 |
| Evaluation of adhesive strength [MPa] | | Before storage | 21.5 | 18.6 | 16.6 | 19.3 | 18.2 | 13.4 | 23.5 | 5.2 | 2.5 | 3.5 |
| | | After storage | 19.4 | 17.5 | 15.1 | 17.2 | 15.7 | 10.8 | 2.3 | 3.1 | 0.8 | 0 |

As shown in Table 1, in examples using a dental adhesive composition containing a (meth)acrylate compound (1) having an acidic group and at least one polymerizable compound (2) selected from the group consisting of a cyclopolymerizable compound (2a) and a polymerizable compound (2b) having a cyclic structure and one polymerizable group, good adhesive strength could be maintained before and after storage. That is, adhesiveness was excellent and storage stability was excellent.

On the other hand, in Comparative Example 2 and Comparative Example 3 not containing the (meth)acrylate compound (1), the adhesive strength was poor before storage, and the adhesive strength before storage could not be maintained after storage.

In Comparative Example 1 containing an adhesive monomer and not containing the polymerizable compound (2), the adhesive strength before storage was excellent, but the adhesive strength before storage could not be maintained after storage.

In Comparative Example 4 containing neither the adhesive monomer nor the polymerizable compound (2), the adhesive strength before storage was poor, and the adhesive strength before storage could not be maintained after storage.

Hereinafter, examples of the second embodiment will be described, but the second embodiment is not limited by the following examples. Abbreviations of compounds used in examples of the second embodiment are shown below.

The abbreviations of compounds used in the present examples are shown below.
3G: triethylene glycol dimethacrylate
HEMA: 2-hydroxyethyl methacrylate
DEMA: decyl methacrylate
TMHDI: mixture of 2,2,4-trimethylhexamethylene diisocyanate and 2,4,4-trimethylhexamethylene diisocyanate
HPA: 2-hydroxypropyl acrylate
XDI: m-xylylene diisocyanate
THIOL: mixture of 5,7-dimercaptomethyl-1,11-dimercapto-3,6,9-trithiaundecane, 4,7-dimercaptomethyl-1,11-dimercapto-3,6,9-trithiaundecane, and 4,8-dimercaptomethyl-1,11-dimercapto-3,6,9-trithiaundecane
DBTDL: dibutyltin dilaurate
BHT: dibutylhydroxytoluene
UDMA: urethane dimethacrylate produced by a production method described below
TUA: thiourethane acrylate produced by a production method described later
TUM: thiourethane methacrylate produced by a production method described later
AOMA: methyl 2-(allyloxymethyl)acrylate
CHA: cyclohexyl acrylate
HPPA: 1-hydroxy-3-phenylpropyl acrylate
PEA: 2-phenoxyethyl acrylate
MDP: 10-(phosphonooxy)decyl methacrylate
PEMA: 2-(phosphonooxy)ethyl methacrylate
4-MET: 4-methacryloxyethyl trimellitic acid
CQ: camphorquinone
DMBE: 2-butoxyethyl 4-(dimethylamino)benzoate
EtOH: ethanol
DW: distilled water

### [Production Example 1: Production of thiourethane acrylate (TUA)]

Into a sufficiently dried 100 mL four-necked flask equipped with a stirring blade and a thermometer, 0.1 parts by weight of DBTDL, 0.05 parts by weight of BHT, 21.35 parts by weight of XDI, and 2.09 parts by weight of THIOL were charged and dissolved to form a homogeneous solution, which was then reacted at 80°C for 4 hours to obtain a solution containing an intermediate. The solution was heated to 90°C, and 26.56 parts by weight of HPA was further added dropwise over 1 hour. Since the internal temperature increased due to the reaction heat during the dropwise addition, the amount of dropwise addition was controlled so as to be 90°C or lower. After dropping the whole amount, the reaction was carried out for 10 hours while the reaction temperature was maintained at 90°C. At this time, the progress of the reaction was followed by HPLC analysis to confirm the end point of the reaction. The product was discharged from the reactor to give 50 g of thiourethane acrylate (TUA).

### [Production Example 2: Production of thiourethane methacrylate (TUM)]

Into a sufficiently dried 100 mL four-necked flask equipped with a stirring blade and a thermometer, 0.1 parts by weight of DBTDL, 0.05 parts by weight of BHT, 22.71 parts by weight of TMHDI, and 1.99 parts by weight of THIOL were charged and dissolved to form a homogeneous solution, which was then reacted at 80°C for 4 hours to obtain a solution containing an intermediate. The solution was heated to 90°C, and 25.30 parts by weight of HEMA was further added dropwise over 1 hour. Since the internal temperature increased due to the reaction heat during the dropwise addition, the amount of dropwise addition was controlled so as to be 90°C or lower. After dropping the whole amount, the reaction was carried out for 10 hours while the reaction temperature was maintained at 90°C. At this time, the progress of the reaction was followed by HPLC analysis to confirm the end point of the reaction. The product was discharged from the reactor to give thiourethane methacrylate (50 g).

### [Production Example 3: Production of urethane dimethacrylate (UDMA)]

Into a sufficiently dried 100 mL four-necked flask equipped with a stirring blade and a thermometer, 0.05 parts by mass of DBTDL, 0.025 parts by mass of BHT, and 22.34 parts by mass of TMHDI were charged and dissolved to form a homogeneous solution, and then this solution was heated to 80°C, and further 27.66 parts by mass of HEMA was added dropwise over 1 hour. Since the internal temperature increased due to the reaction heat during the dropwise addition, the amount of dropwise addition was controlled so as to be 90°C or lower. After the whole amount of HEMA was added dropwise, the reaction was performed for 3 hours while the reaction temperature was maintained at 90°C. At this time, the progress of the reaction was followed by HPLC analysis to confirm the end point of the reaction. The product was discharged from the reactor to obtain 50 g of urethane dimethacrylate.

### [Method of Adhesive Strength Test]

The method of the adhesive strength test in examples and comparative examples is shown below.

### (Preparation of Test Piece for Adhesive Strength Test)

After removal, the cryopreserved cow mandible front teeth were thawed under water injection, and the tooth roots were cut and the pulp was extracted. This was placed in a plastic cylindrical container having a diameter of 25 mm and a depth of 25 mm, and embedded in an acrylic resin. This surface was wet-polished using emery paper of # 120 and # 400, and the dentin was scraped so as to be parallel to the lip surface to prepare a dentin adherend.

Next, the flat surface was dried by blowing compressed air for about 1 second, then the prepared composition was applied to the flat surface of the enamel, and weak blown compressed air was blown. The surface was irradiated with light for 20 seconds using a visible light irradiation device (Translux 2Wave, manufactured by Heraeus Kulzer Japan Co., Ltd.). Furthermore, a plastic mold having a diameter of 2.38 mm (manufactured by ULTRADENT PRODUCTS, INC.) was placed thereon, filled with a dental composite resin (Venus Diamond, manufactured by Heraeus Kulzer Japan Co., Ltd.), and the resin was irradiated with light for 20 seconds using a visible light irradiation device to be cured. Thereafter, the mold was removed to prepare an adhesive sample for dentin.

A porcelain adherend was prepared using a porcelain disk-shaped flat plate (manufactured by VITA Corporation) in place of the cow mandible front teeth. An adhesive sample for a porcelain material was prepared using this adherend.

A gold alloy adherend was prepared using a gold alloy molded product (manufactured by GC) instead of the cow mandible front teeth. An adhesive sample for a gold alloy was prepared using this adherend.

### [Measuring Method in Adhesive Strength Test]

Before storing the sample and after storing the sample in warm water at 37°C for 24 hours, the following adhesive strength test was performed and used as an index of storage stability of the sample.

Using a general-purpose tester (Precision universal material testing machine 210 X, manufactured by INTESCO Co., Ltd.), various adhesion samples were subjected to a shear load at a crosshead speed of 1.0 mm/min in a state of being parallel and in contact with the surface. Then, the adhesive strength was determined from the shear load when the composition formed in a columnar shape on the sample surface was separated from the surface. The values of the obtained adhesive strength are shown in Table 1.

The obtained numerical values were evaluated according to the following evaluation criteria.

### Evaluation Criteria

A: The value of the adhesive strength was 12.5 MPa or more.
B: The value of the adhesive strength was 7.5 MPa or more and less than 12.5 MPa.
C: The value of the adhesive strength was less than 7.5 MPa.

### Example 1A

4.14 parts by mass of UDMA, 0.59 parts by mass of HEMA, 0.59 parts by mass of MDP, 0.59 parts by mass of AOMA, 0.03 parts by mass of CQ, and 0.06 parts by mass of DMBE were put in a container, and stirred at 50°C until they became uniform. Subsequently, 4 g of an aqueous solution adjusted to EtOH : DW = 3 : 2 was added, and the mixture was stirred at normal temperature until the mixture became uniform, thereby obtaining a monomer composition (1). An adhesive strength test was performed according to the methods described in the sections of (Preparation of Test Piece for Adhesive Strength Test) and (Curved Adhesive Strength Test) from the obtained monomer composition (1). The results are shown in Table 2.

### [Examples 2A to 16 A, Comparative Examples 1A to 5A]

A monomer composition (1) was prepared in the same manner as in Example 1A except that the composition of the monomer composition (1) was changed to the composition shown in Table 2, and an adhesive strength test was performed. The results are similarly shown in Table 2.

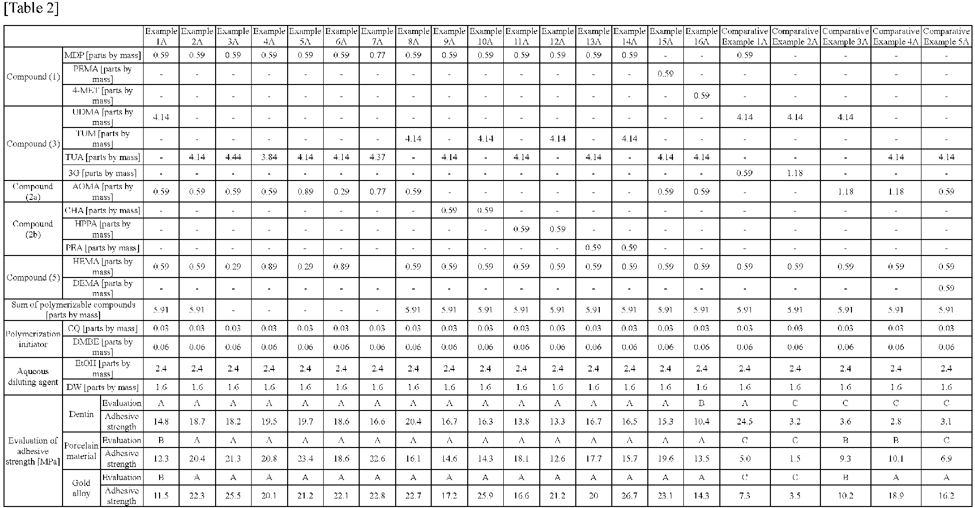

As shown in Table 2, examples using the dental adhesive composition containing the (meth)acrylate compound (1) having an acidic group, at least one polymerizable compound (2) selected from the group consisting of the cyclopolymerizable compound (2a) and the polymerizable compound (2b) having a cyclic structure and one polymerizable group, and the (meth)acrylate compound (3) having two or more (meth)acryloyloxy groups exhibited excellent adhesive strength to dentin and porcelain materials. In addition, the dental adhesive composition according to the example was excellent in adhesive strength to a gold alloy.

On the other hand, Comparative Example 1A containing no polymerizable compound (2) was poor in adhesive strength to a porcelain material and a gold alloy.

Comparative Example 3A and Comparative Example 4A not containing the (meth)acrylate compound (1) were inferior in adhesive strength to dentin.

Comparative Example 2A containing neither the (meth)acrylate compound (1) nor the polymerizable compound (2) was inferior in adhesive strength to all of dentin, porcelain materials, and gold alloys.

Comparative Example 5A not containing the (meth)acrylate compound (1) and containing DEMA, which is a (meth)acrylate compound having no acidic group, was inferior in adhesive strength to dentin and porcelain materials.

The disclosures of Japanese Patent Application No. 2020-118821 filed on July 9, 2020 and Japanese Patent Application No. 2020-118822 filed on July 9, 2020 are incorporated herein by reference in their entirety.

All documents, patent applications, and technical standards described in this specification are incorporated herein by reference to the same extent as if each document, patent application, and technical standard were specifically and individually indicated to be incorporated by reference.

## Claims

1. A dental adhesive composition, comprising:
a (meth)acrylate compound (1) having an acidic group; and
at least one polymerizable compound (2) selected from the group consisting of a cyclopolymerizable compound (2a) and a polymerizable compound (2b) having a cyclic structure and one polymerizable group.

2. The dental adhesive composition according to claim 1, wherein
the cyclopolymerizable compound (2a) comprises an acrylic acid ester compound (2a-1) having one allyloxy group, and
the polymerizable compound (2b) comprises a (meth)acrylate compound (2b-1) having a cyclic structure having 3 to 12 carbon atoms and one (meth)acryloyloxy group.

3. The dental adhesive composition according to claim 1 or 2, wherein
the polymerizable compound (2) comprises at least one selected from the group consisting of a compound represented by the following Formula (2a-1a) and a compound represented by the following Formula (2b-1a): 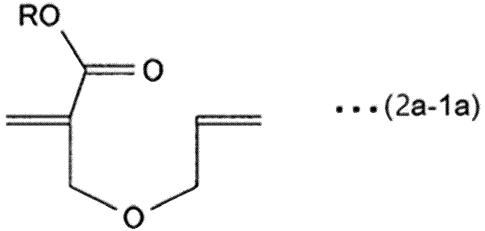
wherein, in Formula (2a-1a), R represents a hydrogen atom or a substituted or unsubstituted hydrocarbon group having 1 to 20 carbon atoms; 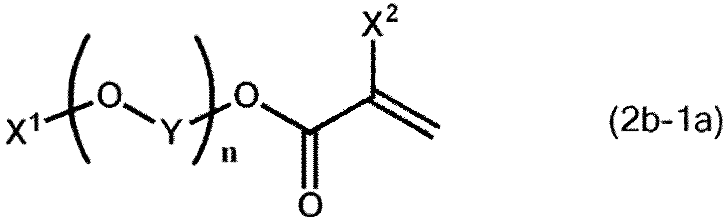
wherein, in Formula (2b-1a), X¹ represents an alicyclic, aromatic, or heterocyclic ring having 4 to 10 carbon atoms, or an alkyl group having 1 to 3 carbon atoms substituted with an alicyclic, aromatic, or heterocyclic ring having 4 to 10 carbon atoms, X² represents a hydrogen atom or a methyl group, Y represents a substituted or unsubstituted alkenyl group having 1 to 5 carbon atoms, and n represents 0 or 1.

4. The dental adhesive composition according to any one of claims 1 to 3, wherein the acidic group in the (meth)acrylate compound (1) comprises a phosphoric acid group.

5. The dental adhesive composition according to any one of claims 1 to 4, wherein a content of the (meth)acrylate compound (1) is 1 part by mass to 20 parts by mass with respect to 100 parts by mass of a sum of polymerizable compounds contained in the dental adhesive composition.

6. The dental adhesive composition according to any one of claims 1 to 5, wherein a content of the polymerizable compound (2) is 1 part by mass to 40 parts by mass with respect to 100 parts by mass of a sum of polymerizable compounds contained in the dental adhesive composition.

7. The dental adhesive composition according to any one of claims 1 to 6, wherein a total content of the (meth)acrylate compound (1) and the polymerizable compound (2) is 1% by mass to 40% by mass with respect to a total mass of the dental adhesive composition.

8. The dental adhesive composition according to any one of claims 1 to 7, further comprising a (meth)acrylate compound (3) having two or more (meth)acryloyloxy groups.

9. The dental adhesive composition according to claim 8, wherein the (meth)acrylate compound (3) comprises at least one selected from the group consisting of a (meth)acrylate compound having two or more urethane bonds and a (meth)acrylate compound having two or more thiourethane bonds.

10. The dental adhesive composition according to claim 8 or 9, wherein the (meth)acrylate compound (3) comprises a reaction product of a thiol compound having two or more thiol groups, an iso(thio)cyanate compound having two or more iso(thio)cyanate groups, and a (meth)acrylate compound (4) having a hydroxy group.

11. The dental adhesive composition according to any one of claims 8 to 10, wherein a content of the (meth)acrylate compound (3) is 10 parts by mass to 95 parts by mass with respect to 100 parts by mass of a sum of polymerizable compounds contained in the dental adhesive composition.

12. The dental adhesive composition according to any one of claims 8 to 11, wherein a total content of the (meth)acrylate compound (1), the polymerizable compound (2), and the (meth)acrylate compound (3) is 30% by mass to 90% by mass with respect to a total mass of the dental adhesive composition.

13. The dental adhesive composition according to any one of claims 1 to 12, comprising an organic solvent and water.

14. The dental adhesive composition according to claim 13, wherein a total content of the organic solvent and the water is 20% by mass to 70% by mass with respect to a total mass of the dental adhesive composition.

15. The dental adhesive composition according to any one of claims 1 to 14, wherein a content of a silane coupling agent is less than 0.10% by mass with respect to a total mass of the dental adhesive composition.

16. The dental adhesive composition according to any one of claims 1 to 15, further comprising a polymerization initiator.

17. The dental adhesive composition according to any one of claims 1 to 16, which is a dental bonding material.

18. A dental material comprising a cured product of the dental adhesive composition according to any one of claims 1 to 17.
